# EUROPEAN PATENT APPLICATION

(11) **EP 2 505 584 A1**
(43) Date of publication of application: **03.10.2012**
(21) Application number: 10833197.6
(22) Date of filing: 24.11.2010
(51) Int. Cl.: C07D 263/58, C07D 277/82

(54) **METHOD FOR PRODUCING CYCLOHEXANE DERIVATIVE**

(30) Priority: 25.11.2009 JP 2009266988
(71) Applicant: Shionogi & Co., Ltd., Osaka 541-0045 (JP)
(72) Inventor: KUNITANI, Ryosuke, Amagasaki-shi Hyogo 660-0813 (JP); TAKAYA, Kenji, Toyonaka-shi Osaka 561-0825 (JP); IMAMURA, Yoshiaki, Amagasaki-shi Hyogo 660-0813 (JP); HASEGAWA, Aiko, Amagasaki-shi Hyogo 660-0813 (JP)
(74) Representative: Vossius & Partner
(86) International application number: PCT/JP2010/070870
(87) International publication number: WO 2011/065351

(57) **Abstract**

A process for the preparation of a compound of formula (I): 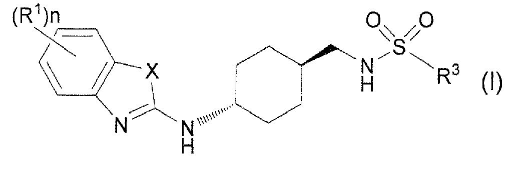 wherein R¹ is each independently halogen, C1-C6 alkyl, C1-C6 alkoxy, C1-C6 haloalkyl, C1-C6 haloalkoxy or C1-C6 alkylcarbonyl,
R³ is C1-C6 alkyl; C3-C8 cycloalkyl; or phenyl optionally substituted with one or more substituents selected from the group consisting of halogen, C1-C6 alkyl; C1-C6 alkoxy, C1-C6 haloalkyl and C1-C6 haloalkoxy,
n is an integer of 0 to 2,
X is S atom or O atom,

its salt or solvate thereof characterized by reacting a compound of formula (IV): 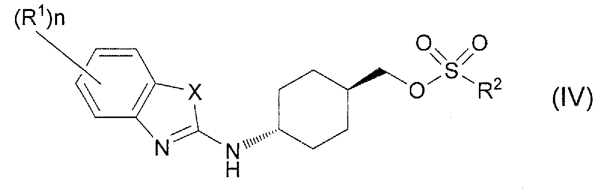 wherein R¹, n and X have the same meaning as defined above, and
R² is C1-C6 alkyl; C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro,

with a compound of formula (V): 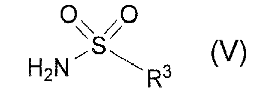 wherein R³ has the same meaning as defined above.

## Description

### Technical Field

This invention relates to a process for the preparation of cyclohexane derivatives, more particularly to a process for the preparation of the compounds having NPYY5 receptor antagonistic activity.

### Background Art

Patent Documents 1 to 3 disclose a compound of formula (I): 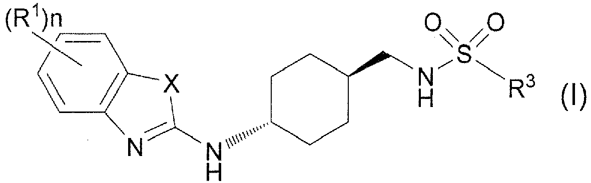 wherein
R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom, and
R³ is C1-C6 alkyl; C3-C8 cycloalkyl; or phenyl optionally substituted with one or more substituents selected from the group consisting of halogen, C1-C6 alkyl, C1-C6 alkoxy, C1-C6 haloalkyl and C1-C6 haloalkoxy,
having an NPY Y5 receptor antagonistic activity.

Patent Documents 1 and 2 describe a compound of the general formula: 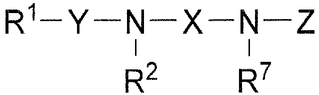 Those documents describe the following scheme as a general description of the procedures for the compounds wherein Y is SO₂:

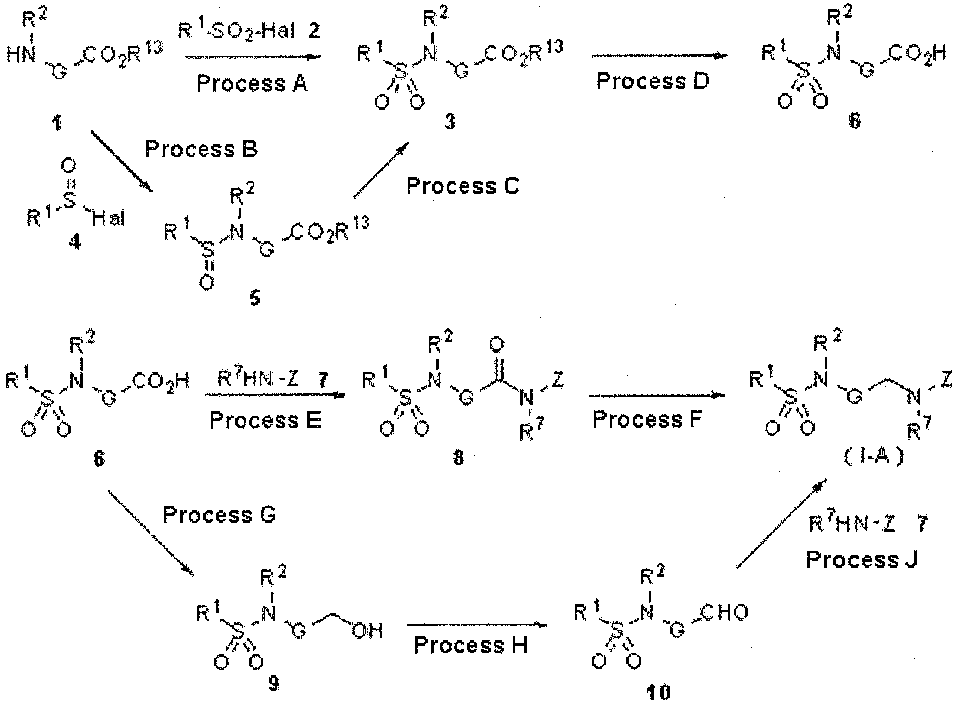

The above general procedures comprise the preparation of the above Compound 3 by the process A at first, reacting the amine (the above Compound 1) with the sulfonylating reagent (the above Compound 2), or by the process B and the process C at first, and then the introduction of the substituent Z by the process D, E and F or by the process D, G, H and J. The present invention of the process for the preparation of a compound of formula (I) is different from the above the general procedures. Patent Documents 1 and 2 do not disclose any specific example corresponding to the above general procedures.

In addition, Patent Documents 1 and 2 disclose the following scheme as a general description of the procedures:

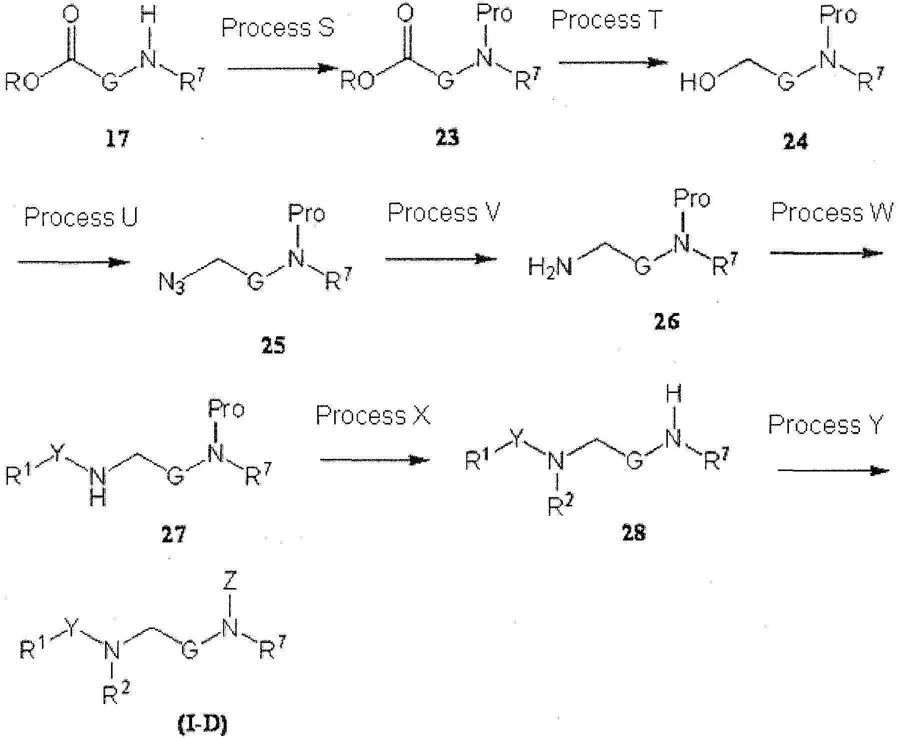

The above general procedures comprise the process W, reacting the amine with the sulfonylating reagent at first, and the process X and Y, then introducing the substituent Z. The present invention of the process for the preparation of the compound of formula (I) is different from the above the general procedures. Patent Documents 1 and 2 disclose Example 3 as a specific example corresponding to the above general procedures.

In addition, Patent Documents 1 and 2 disclose the following scheme as a general description of the process:

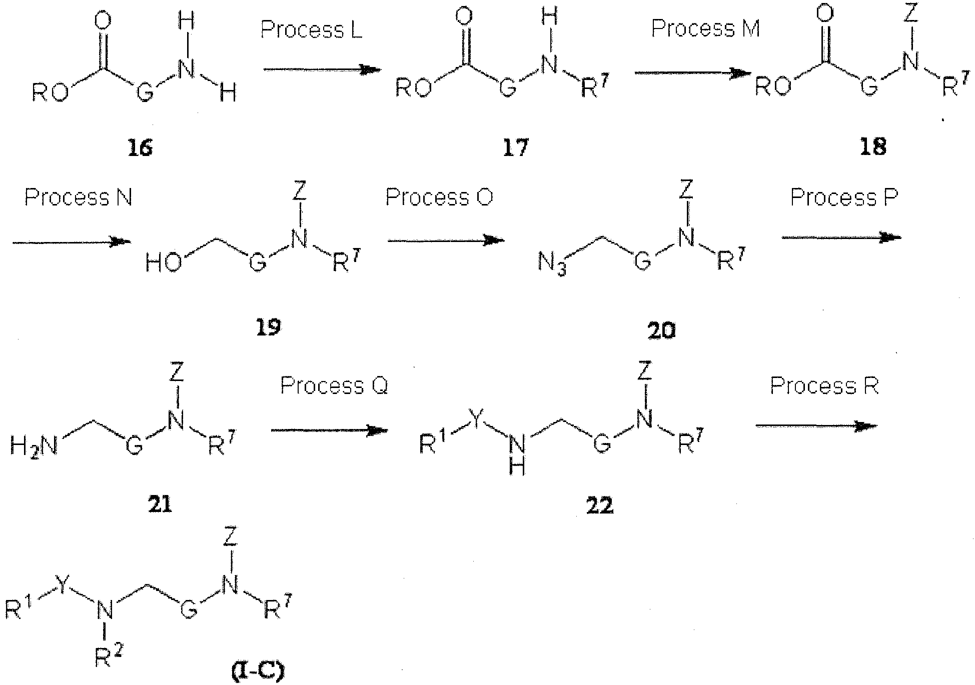

The above general procedures comprise the process M, introducing the substituent Z at first, and the process Q, then reacting the amine with the sulfonylating reagent. The present invention of the process for the preparation of the compound of formula (I) is different from the above the general procedures. Patent Documents 1 and 2 disclose Example 2 as a specific example corresponding to the above general procedures. The target compound is obtained in 29% yields in the following process 3. In addition, the yield in the following process 1 is 20%, and the yield in the following process 2 is 87%.

### Process 1

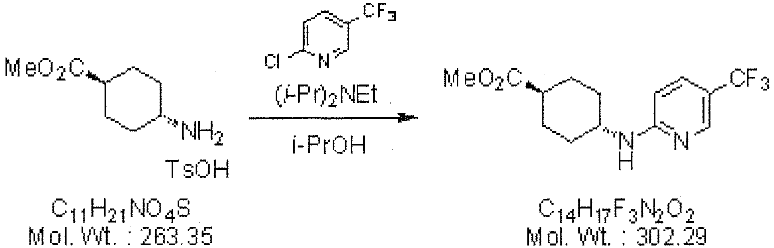

### Process 2

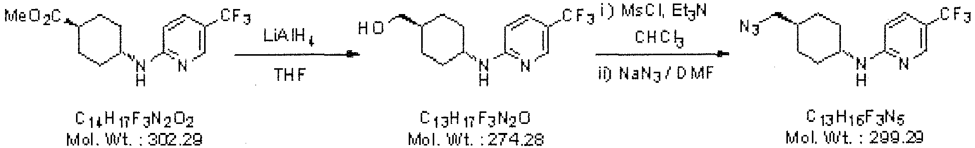

### Process 3

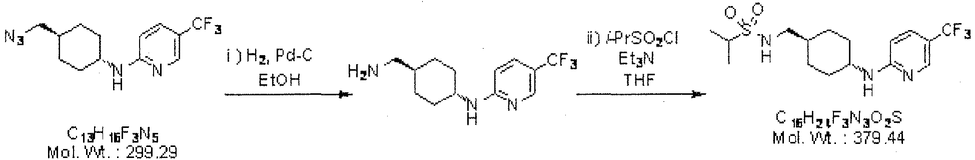

Patent Document 3 discloses a compound of general formula: 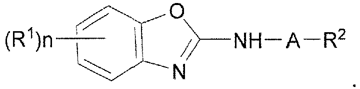 Patent Document 3 discloses the following scheme as a general description of the procedures:

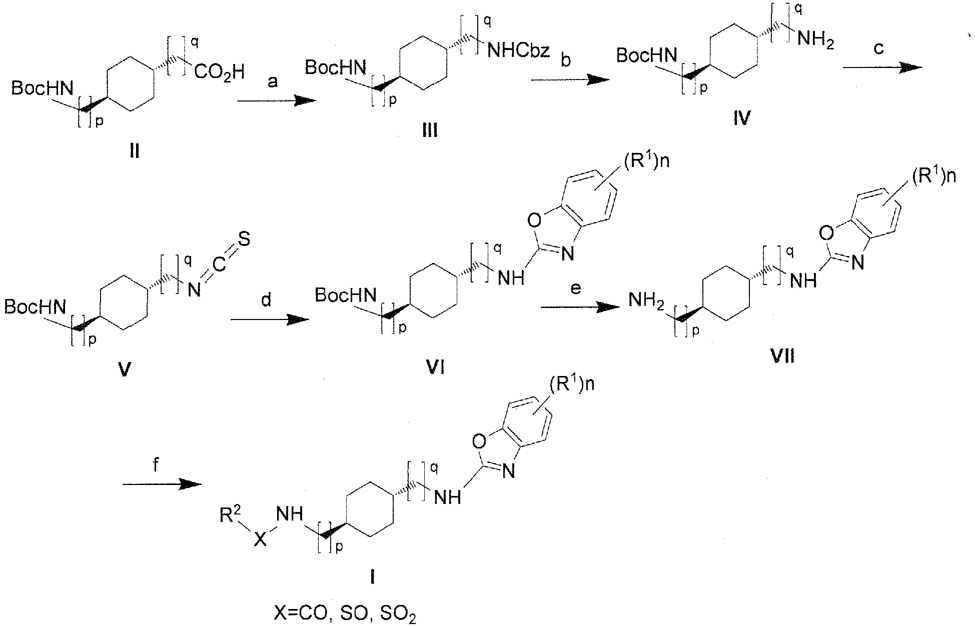

The above general procedures comprise the preparation of the target Compound I by reacting the amine Compound VII with the sulfonylating reagent after the preparation of Compound VI by introducing the benzoxazole part to Compound V. The present invention of the process for the preparation of the compound of formula (I) is different from the above the general procedures.
Patent Document 3 discloses the Example 1 as a specific example corresponding to the above general procedures that Compound VII is converted into the target Compound I.
The target compound is obtained in 66% yields in the process.

The process for the preparation of the compound of formula (I), specially the process for the preparation of the compound of formula (I) by using the compound of formula (IV) as an intermediate, is not described or suggested in Patent Documents 1 to 3.

### Prior Art Documents

### Patent Documents

[Patent Document 1] WO2007/125952
[Patent Document 2] WO2009/054434
[Patent Document 3] WO2008/134228

### Non-Patent Documents

[Non-Patent Document 1] Journal of Organic Chemistry, 2002, 67, 6001-6007
[Non-Patent Document 2] SYNTHESIS, 2006, No. 16, pp2760-2766
[Non-Patent Document 3] Japan process chemistry 2009 Summer symposium Summary p94-95

### Disclosure of Invention

### Problems to be solved by the Invention

The present invention provides a novel and efficient process for the preparation of cyclohexane derivatives of the formula (I).

### Means for Solving the Problem

The example 2 described in Patent Document 1 or 2 is related to the method for the preparation of the sulfonamide from alcohol derivatives via amine derivatives. However, the process of the above preparation has more steps than that of the present invention and the yield is much worse. In addition, the above process for the preparation includes the Step O, the preparation of Compound 20 by azidation of Compound 19. Since a sodium azide necessary for the azidation as a reagent or Compound 20 obtained as a result is explosive, in the view of human body and environment, the above process is not suitable for an industrial preparation of the medicine. The process of the preparation described in Patent Document 3 is different from that of the present invention and does not include any use of alcohol derivatives (the compound of formula (II) described in this specification). In the process of the introduction of benzoxazole part or the reaction of using sulfonylating reagent, amine derivatives (compound IV and compound VII described in Patent Document 3) is used. It is necessary for another amino group to be protected in addition reaction of benzoxazole in the first reaction, lest two amino groups substituted on cyclohexane derivatives (compound II describe in Patent Document 3) are reacted at the same time. As a result, the total steps are long, and the total yield is bad.
The present inventors have achieved to find a process for the preparation of cyclohexane derivatives, that is, a process for the preparation of a compound of formula (I) via a compound of formula (IV) as an intermediate. The processes are different in that the number of process is short, that the explosive reagent is not used, and that the yield is good. Therefore, COGS (cost of goods sold) of the present invention is excellent, the present invention is suitable for industrial use. A compound of formula (IV) is a useful compound as an intermediate. A compound of formula (I) can be prepared via the intermediate effectively, using an explosive reagent can be avoided.

This invention includes the followings.
(1) A process for the preparation of a compound of formula (I): 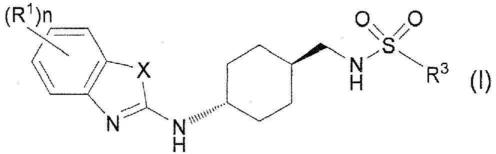 wherein
   R¹ is each independently halogen, C1-C6 alkyl, C1-C6 alkoxy, C1-C6 haloalkyl, C1-C6 haloalkoxy or C1-C6 alkylcarbonyl,
   R³ is C1-C6 alkyl; C3-C8 cycloalkyl; or phenyl optionally substituted with one or more substituents selected from the group consisting of halogen, C1-C6 alkyl; C1-C6 alkoxy, C1-C6 haloalkyl and C1-C6 haloalkoxy,
   n is an integer of 0 to 2, and
   X is S atom or O atom,
   its salt or solvate thereof, characterized by reacting a compound of formula (IV): 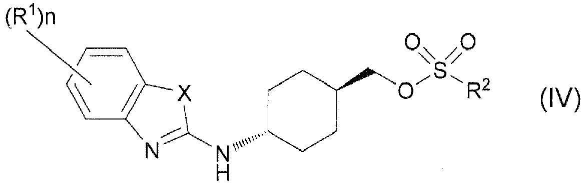 wherein
   R¹, n and X have the same meaning as defined above, and
   R² is C1-C6 alkyl; C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro,
   with a compound of formula (V): 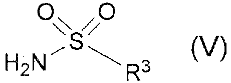 wherein R³ has the same meaning as defined above.
(2) The process for the preparation of the above (1), characterized by reacting a compound of formula (IV) with a compound of formula (V) in the presence of a base.
(3) The process for the preparation of the above (2), wherein the base is alkali metal alkoxide or inorganic carbonate.
(4) The process for the preparation of the above (3), wherein the base is potassium tert-butoxide, sodium tert-butoxide, sodium methoxide, sodium ethoxide, sodium pentoxide, sodium carbonate, potassium carbonate, calcium carbonate or cesium carbonate.
(5) The process for the preparation of the above (2), characterized by reacting in the polar solvent.
(6) The process for the preparation of the above (5), wherein the polar solvent is one or more solvents selected from the group consisting of methanol, ethanol, isopropanol, n-propanol, tert-butanol, n-butanol, s-butanol, N, N-dimethylformamide, N, N-dimethylacetoamide, N-methylpyrrolidone, 1, 3-dimethyl-2-imidazolidinone, ethyl acetate, propyl acetate, tetrahydrofuran, 2-methyl tetrahydrofuran, dimethylsulfoxide, acetonitrile, propyonitrile, acetone, methylethylketone and methylisobutylketone.
(7) The process for the preparation according to any one of (1) to (6), wherein R² is methyl.
(8) The process for the preparation according to any one of the above (1) to (7), including the process for the preparation of a compound of formula (IV): 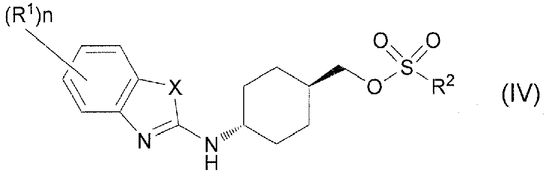
   wherein R¹ is each independently halogen, C1-C6 alkyl, C1-C6 alkoxy, C1-C6 haloalkyl, C1-C6 haloalkoxy or C1-C6 alkylcarbonyl,
   R² is C1-C6 alkyl; C1-C6 haloalkyl or phenyl optionally substituted with one or more substituents selected from the group consisting of halogen, C1-C6 alkyl, C1-C6 alkoxy, C1-C6 haloalkyl, C1-C6 haloalkoxy and nitro,
   n is an integer of 0 to 2,
   X is S atom or O atom,
   its salt or solvate thereof,
   comprising reacting a compound of formula (II): 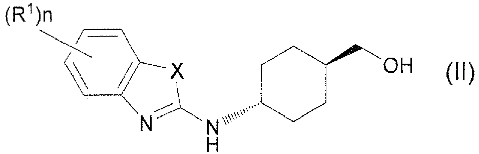
   wherein R¹, n and X have the same meaning in a compound of formula (IV), with a compound of formula (III):

      R²-SO₂-Y

      wherein R² has the same meaning in a compound of formula (IV), and
      Y is a leaving group.
(9) The process for the preparation of the above (8), characterized by reacting a compound of formula (II) with a compound of formula (III) in the presence of a base.
(10) The process for the preparation of the above (9), wherein the base is an organic base.
(11) The process for the preparation of the above (10), wherein the base is triethylamine, dimethylaminopyridine, diazabicycloundecene, diisopropylethylamine, N-methyl imidazole or N-methylmorpholine.
(12) The process for the preparation according to any one of the above (9) to (11), characterized by using 2mol to 5mol equivalents of the base to the compound (II).
(13) The process for the preparation of the above (8), characterized in reacting a compound of formula (II) with a compound of formula (III) in one or more solvents selected from the group consisting of polar solvent, toluene and dichloromethane.
(14) The process for the preparation of the above (14), wherein the polar solvent is one or more polar solvents selected from the group consisting of N, N-dimethylformamide, N, N-dimethylacetamide, N-methylpyrrolidone, 1,3-dimethyl-2-imidazolidinone, ethyl acetate, propyl acetate, cyclopentylmethylether, tetrahydrofuran, 2-methyltetrahydrofuran, dimethylsulfoxide, acetonitrile, propionitrile and methyl isobutyl ketone.
(15) The process for the preparation according to any one of the above (8) to (14), characterized by not isolating nor purifying a compound of formula (IV), its salt or solvate thereof.
(16) The process for the preparation according to any one of the above (8) to (15), characterized by using 2mol to 5mol equivalents of the compound (III) to the compound (II).
(17) The process for the preparation of a compound of formula (I): 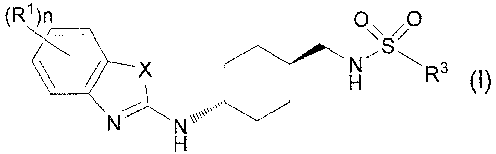
   wherein R¹ is each independently halogen, C1-C6 alkyl, C1-C6 alkoxy, C1-C6 haloalkyl, C1-C6 haloalkoxy or C1-C6 alkylcarbonyl,
   R³ is C1-C6 alkyl; C3-C8 cycloalkyl; or phenyl optionally substituted with one or more substituents selected from the group consisting of halogen, C1-C6 alkyl; C1-C6 alkoxy, C1-C6 haloalkyl and C1-C6 haloalkoxy,
   n is an integer of 0 to 2, and
   X is S atom or O atom,
   its salt or solvate thereof,
   characterized by reacting a compound of formula (II): 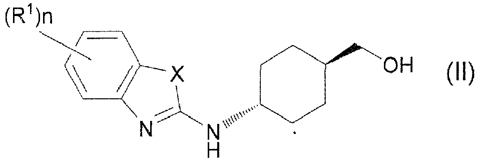
   wherein R¹, n and X have the same meaning in a compound of formula (I), with a compound of formula (III):

      R²-SO₂-Y

      wherein R² is C1-C6 alkyl; C1-C6 haloalkyl or phenyl optionally substituted with one or more substituents selected from the group consisting of halogen, C1-C6 alkyl, C1-C6 alkoxy, C1-C6 haloalkyl, C1-C6 haloalkoxy and nitro,
      Y is a leaving group,
   to obtain a compound of formula (IV): 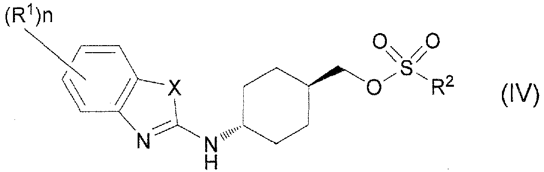 wherein R¹, R², n and X have the same meaning in a compound of formula (II) or (III),
   and reacting the obtained compound (IV) with a compound of formula (V): 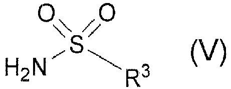 R³ has the same meaning in a compound of formula (I).
(18) A compound of formula (II): 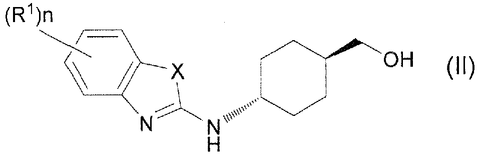
   wherein R¹ is each independently halogen, C1-C6 alkyl, C1-C6 alkoxy, C1-C6 haloalkyl, C1-C6 haloalkoxy or C1-C6 alkylcarbonyl,
   n is an integer of 0 to 2, and
   X is S atom or O atom,
   its salt or solvate thereof.
(19) The compound, its salt or solvate thereof of the above (18),
   wherein R¹ is fluorine or chlorine, and
   n is an integer of 0 or 1.
(20) A compound of formula (IV): 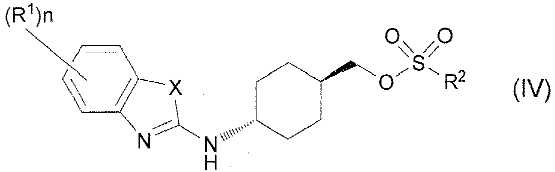
   wherein R¹ is each independently halogen, C1-C6 alkyl, C1-C6 alkoxy, C1-C6 haloalkyl, C1-C6 haloalkoxy or C1-C6 alkylcarbonyl,
   n is an integer of 0 to 2,
   X is S atom or O atom, and
   R² is C1-C6 alkyl; C1-C6 haloalkyl or phenyl optionally substituted with one or more substituents selected from the group consisting of halogen, C1-C6 alkyl, C1-C6 alkoxy, C1-C6 haloalkyl, C1-C6 haloalkoxy and nitro,
   its salt or solvate thereof.
(21) The compound, its salt or solvate thereof of the above (20),
   wherein R¹ is fluorine or chlorine, and
   n is an integer of 0 or 1.
(22) The compound, its salt or solvate thereof of the above (20),
   wherein R² is methyl.
(23) A process for the preparation of a compound of formula (IV): 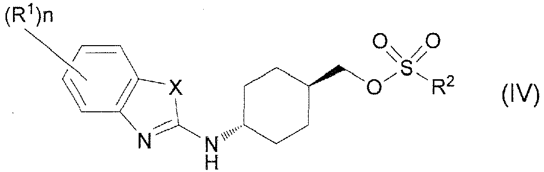
   wherein R¹ is each independently halogen, C1-C6 alkyl, C1-C6 alkoxy, C1-C6 haloalkyl, C1-C6 haloalkoxy or C1-C6 alkylcarbonyl,
   R² is C1-C6 alkyl; C1-C6 haloalkyl or phenyl optionally substituted with one or more substituents selected from the group consisting of halogen, C1-C6 alkyl, C1-C6 alkoxy, C1-C6 haloalkyl, C1-C6 haloalkoxy and nitro,
   n is an integer of 0 to 2, and
   X is S atom or O atom,
   its salt or solvate thereof characterized by reacting a compound of formula (II): 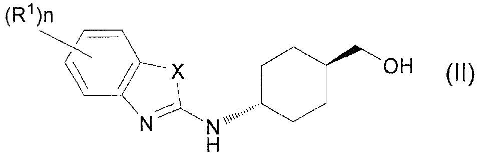
   wherein R¹ and n have the same meaning in a compound of formula (IV), with a compound of formula (III):

      R²-SO₂-Y

      wherein R² has the same meaning in a compound of formula (IV), and
      Y is a leaving group.
(24) The process for the preparation of the above (23), characterized by using 2mol to 5mol equivalents of the compound (III) to the compound (II).
(25) The process for the preparation of the above (23) or (24), characterized by reacting a compound of formula (II) with a compound of formula (III) in the presence of 2mol to 5mol equivalents of the base to the compound (II).
(26) A process for the preparation of a compound of formula (II): 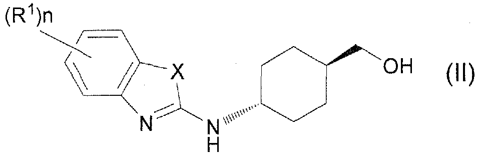
   wherein R¹ is each independently halogen, C1-C6 alkyl, C1-C6 alkoxy, C1-C6 haloalkyl, C1-C6 haloalkoxy or C1-C6 alkylcarbonyl,
   n is an integer of 0 to 2, and
   X is S atom or O atom,
   its salt or solvate thereof, characterized by reacting a calcium chloride and NaBH₄ with a compound of formula (B): 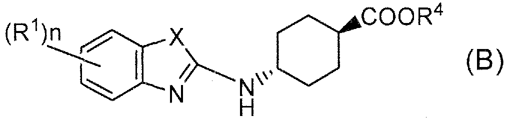
   wherein R¹ and n have the same meaning as defined above,
   R⁴ is ester residue.

### Effect of the Invention

A process for the preparation of the present invention can be used to prepare Compound (I) effectively.

### Best Mode for Carrying Out the Invention

Terms used in the present description are explained below.

"Halogen" includes fluorine, chlorine, bromine and iodine. Especially preferred is fluorine or chlorine.

"C1-C6 alkyl" includes C1 to C6 straight or branched alkyl. It includes C1 to C4 alkyl, C1 to C3 alkyl and the like. Examples include methyl, ethyl, n-propyl, isopropyl, n-butyl, isobutyl, see-butyl, tert-butyl, n-pentyl, isopentyl, neopentyl, hexyl, isohexyl and the like.

"C1-C6 alkoxy" means C1-C6 alkyl wherein the C1-C6 alkyl is bonded to an oxygen atom. Examples include methoxy, ethoxy, n-propoxy, isopropoxy, n-butoxy, isobutoxy, sec-butoxy, tert-butoxy, n-pentoxy, pentoxy, neopentoxy, hexoxy, isohexyoxy and the like.

"'C1-C6 Haloalkyl" and "C1-C6 haloalkyloxy" means "C1-C6 alkyl" and "C1-C6 alkoxy" wherein is "C1-C6 alkyl" and "C1-C6 alkoxy" is substituted with the above "halogen". The number of "halogen" is not limited, and preferred is 1 to 5.

"C1-C6 alkyloxycarbonyl" means the above "C1-C6 alkyl" is bonded to a carbonyl.

"C3-C8 cycloalkyl" means C3 to C8 cyclic alkyl. It included C3-C6 cyclic alkyl, C5 or C6 cyclic alkyl and the like. Examples include cyclopropyl, cyclobutyl, cyclopentyl, cyclohexyl, cycloheptyl, cyclooctyl and the like.

"Phenyl optionally with one or more substituents selected from the group consisting of halogen, C1-C6 alkyl, C1-C6 alkoxy, C1-C6 haloalkoxy and nitro" or "phenyl optionally with one or more substituents selected from the group consisting of halogen, C1-C6 alkyl, C1-C6 alkoxy and C1-C6 haloalkoxy" means the phenyl optionally substituted with the substituent(s) at arbitrary position(s). Preferable example includes phenyl optionally substituted with the 1 to 3 substituents, moreover preferable is phenyl optionally substituted with the 1 to 2 substituents. When the phenyl is substituted with a number of substituents, the each substituent is can be same or different.

A leaving group is not limited as long as it efficiently leaves in the sulfonylation of alcohol. Examples of a leaving group include halogen, formula: -O-SO₂-R² (wherein R² is C1-C6 alkyl C1-C6 haloalkyl or phenyl optionally substituted with one or more substituents selected from the group consisting of halogen, C1-C6 alkyl, C1-C6 alkoxy, C1-C6 haloalkyl, C1-C6 haloalkoxy and nitro. Preferable example includes halogen. Moreover preferable example includes chloro.

Examples of salt include salts with inorganic acids such as hydrochloric acid, sulfuric acid, nitric acid, phosphoric acid and the like; and organic acids such as acetic acid, formic acid, p-toluenesulfonic acid, methanesulfonic acid, oxalic acid, citric acid and the like.

"Solvate" includes a hydrate, an alcohol solvate and the like of a compound or its salt. Examples of solvate are 1 hydrate, 2 hydrates, 1 alcohol solvate, 2 alcohols, a solvate of a compound or its salt.

Any "ester residue" can be used as long as formula:-C(=O)=OR⁴ is reduced with NaBH₄ to convert alcohol. A preferable embodiment of R⁴ includes C1-C6 alkyl; C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro and the like.

Reaction of a compound with a compound includes reaction of salt of the each compound or solvate thereof in the present description.

The process of the present invention can be conducted as follows:

### Process 1

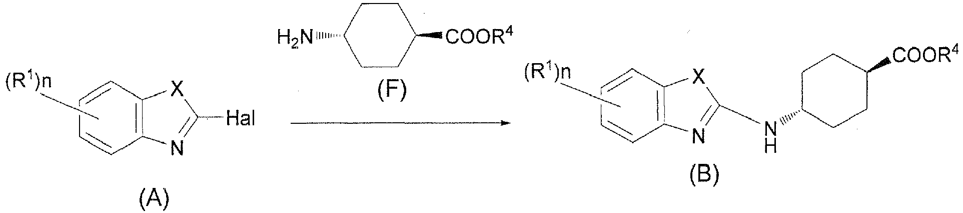

wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom,
Hal is halogen, and
R⁴ is ester residue.
The compound of formula (B) can be prepared by reacting Compound (A) with Compound (F) and a base in solvent.
A base is not limited as long as it efficiently proceeds in the above process. Organic base or inorganic base such as inorganic carbonate and the like can be used. Organic base can be used preferably. Examples of a base include triethylamine, pyridine, dimethyaminopyridine, diazabicycloundecene, 1, 8-bis (dimethylamino) naphthalene, diisopropylethylamine, N-methyl imidazole and N-methylmorpholine and the like. Especially preferable example of a base includes triethylamine.
The amount of the base can be 1 mol to 5mol equivalent(s) to Compound (F).
A solvent is not limited as long as it efficiently proceeds in the above process. One or more solvents selected from the group consisting of methanol, ethanol, isopropanol, 1, 2-dimethoxyethane, N, N-dimethylformamide, N,N-dimethylacetoamide, N-methyl pyrrolidone, 1,3-dimethyl-2-imidazolidinone, ethyl acetate, propyl acetate, toluene, cyclopentylmethylether, tetrahydrofuran, 2-methyl tetrahydrofuran, dimethylsulfoxide, acetonitrile, propionitrile and the like can be used. The solvent(s) can be used two phase solvents with water or hydrous solvent, if necessary. Preferable solvent includes polar solvent.
Examples of polar solvents include one or more solvents selected from the group consisting of methanol, ethanol, isopropanol, 1, 2-dimethoxyehane, N, N-dimethylformamide, N,N-dimethylacetoamide, N-methyl pyrrolidone, 1,3-dimethyl-2-imidazolidinone, ethyl acetate, propyl acetate, cyclopentylmethylether, tetrahydrofuran, 2-methyl tetrahydrofuran, dimethylsulfoxide, acetonitrile, propionitrile and the like. Preferable examples include one or more solvents selected from the group consisting of 1,2 -dimethoxyethane, N, N-dimethylformamide, N,N-dimethylacetoamide, N-methyl pyrrolidone, 1,3-dimethyl-2-imidazolidinone, ethyl acetate, propyl acetate, cyclopentylmethylether, tetrahydrofuran, 2-methyl tetrahydrofuran, dimethylsulfoxide, acetonitrile, propionitrile and the like. Especially preferable example includes N, N-dimethylformamide.
The temperature for such reaction is not limited, but usually can be about 0 to 100 °C and preferably about room temperature to 70 °C.
Reaction time is not limited, but usually can be conducted for 0.5 to 20 hours and preferably 1 to 10 hour(s).

The obtained compound (B) also includes its salt or solvate thereof.
The compound of formula (B): 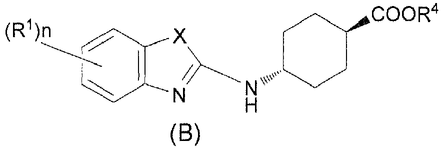
wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom, and
R⁴ is ester residue,
its salt or solvate thereof is exemplified.
   R¹ is preferably fluorine or chlorine.
   n is preferably 0 or 1.
   R⁴ is preferably ethyl.

### Process 2

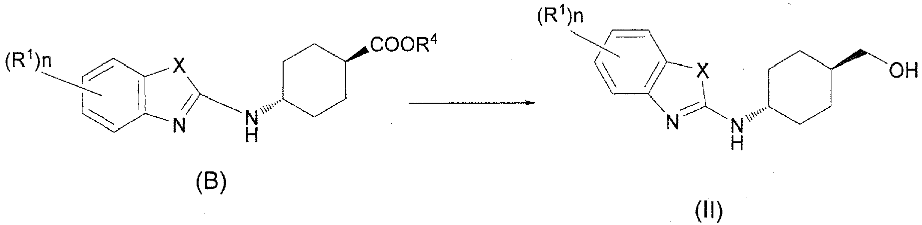

wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom, and
R⁴ is ester residue.
The compound of formula (II) can be prepared by reducing Compound (B).
A reducing reagent is not limited as long as it efficiently proceeds in the above process. Examples of a reducing reagent include lithium aluminum hydride, sodium borohydride, lithium borohydride, borane and the like. Preferable examples include lithium borohydride or sodium borohydride. Moreover, preferable example includes sodium borohydride.
The amount of the reducing reagent can be 1mol to 5mol equivalent(s) to Compound (B).
Catalyst may be added, if necessary. A reactivity of a reducing reagent can be enhanced by adding a catalyst. As a result, the amount of the reducing reagent can be reduced. The amount of the reducing reagent can be 1mol to 3mol equivalent(s) to Compound (B) by adding a catalyst. As a result, COGS gets good by reducing the amount of the using reagent, it is very suitable for industrial use. Preferable example of catalyst includes calcium chloride.
A solvent is not limited as long as it efficiently proceeds in the above process. One or more solvents selected from the group consisting of methanol, ethanol, isopropanol, n-propanol, tert-butanol, n-butanol, 1,2- dimethoxyethane, N, N-dimethylformamide, N,N-dimethylacetoamide, N-methyl pyrrolidone, 1,3-dimethyl-2-imidazolidinone, toluene, cyclopentylmethylether, tetrahydrofuran, 2-methyl tetrahydrofuran, dimethylsulfoxide and the like can be used. The solvent can be used two phase solvents with water or hydrous solvent, if necessary. Preferable solvent includes polar solvent.
Examples of polar solvent include one or more solvents selected from the group consisting of methanol, ethanol, isopropanol, n-propanol, tert-butanol, n-butanol, 1,2-dimethoxyethane, N, N-dimethylformamide, N,N-dimethylacetoamide, N-methyl pyrrolidone, 1,3-dimethyl-2-imidazolidinone, cyclopentylmethylether, tetrahydrofuran, 2-methyl tetrahydrofuran, dimethylsulfoxide and the like. Preferable examples are mixed solvent with tetrahydrofuran and methanol.
The temperature for such reaction is not limited, but usually can be about 0 to 100 °C and preferably about room temperature to 80 °C. When calcium chloride is used as a catalyst, the temperature for such reaction can be room temperature to about 50°C as described below Example 4-2. The reaction solution need not be boiled. When the quantity synthesis is conducted, since it is easy and safe to control the heat of the reaction, it is very suitable for industrial use.
Reaction time is not limited, but usually can be conducted for 0.5 to 20 hours and preferably 1 to 10 hour(s).
When the reducing reagent is added, it is preferable that the reducing reagent is dissolved in solvent, and that the solution is added dropwise. Compound (B) can be reacted with the reducing reagent at the same time as being added dropwise, the reaction heat and the rate of gas evolution can be controlled safely. If Compound (B) is not reacted with the reducing reagent at the same time as being added dropwise, it is difficult to control the evolved heat and the gas evolution and it is danger to conduct the quantity synthesis for industrial use.

The obtained compound (II) also includes the salt or solvate thereof.
The compound of formula (II): 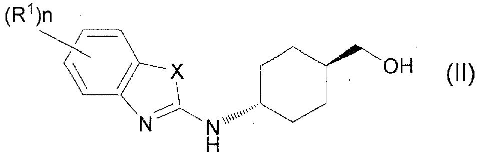
wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2, and
X is S atom or O atom,
its salt or solvate thereof is exemplified.
R¹ is preferably fluorine or chlorine.
n is preferably 0 or 1.
The compound of formula (II) obtained can be precipitated in aqueous acetone to obtain as a solid. When a calcium chloride as a catalyst is used in the Process 2, a very insoluble substance is precipitated at the same time as the compound of formula (II) is precipitated. The precipitation of the very insoluble substance is suppressed by adding propionic acid.
The Process 2 and the process of precipitation of the compound of formula (II) can be conducted in one-pot method. Since it is not necessary to be extracted, concentrated, purified with columns and the like, it is very suitable for industrial use.

### Process 3

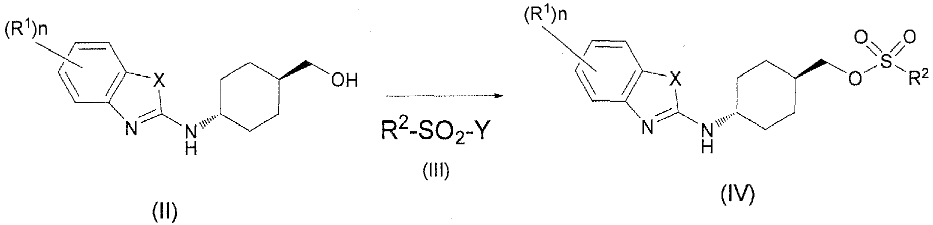

wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom,
R² is C1-C6 alkyl; C1-C6 haloalkyl or phenyl optionally substituted with one or more substituents selected from the group consisting of halogen, C1-C6 alkyl, C1-C6 alkoxy, C1-C6 haloalkyl, C1-C6 haloalkoxy and nitro, and
Y is a leaving group.
A compound of formula (IV) can be prepared by reacting a compound of formula (II) with a compound of formula (III) in the presence of a base.
A base is not limited as long as it efficiently proceeds in the above process. Organic base or inorganic base such as inorganic carbonate and the like can be used. Organic base can be used preferably. Examples of a base include triethylamine, pyridine, dimethyaminopyridine, diazabicycloundecene, 1, 8-bis (dimethylamino) naphthalene, diisopropylethylamine, N-methyl imidazole, N-methylmorpholine and the like. Moreover, preferable examples of a base include triethylamine, dimethyaminopyridine, diazabicycloundecene, diisopropylethylamine, N-methyl imidazole or N-methylmorpholine. Especially preferable example of a base includes triethylamine.
The amount of the base can be 1mol to 5mol equivalent(s) to Compound (II). The amount of the base can be especially preferably more than 1.5mol equivalents, moreover preferably more than 2mol equivalents to Compound (II).
The amount of the compound of formula (III) can be 1mol to 5mol equivalent(s) to Compound (II). The amount of a compound of formula (III) can be especially preferably more than 1.5mol equivalents, moreover preferably more than 2mol equivalents to Compound (II).
A solvent is not limited as long as it efficiently proceeds in the above process. One or more solvents selected from the group consisting of 1,2-dimethoxyethane, N, N-dimethylformamide, N,N-dimethylacetoamide, N-methyl pyrrolidone, 1,3-dimethyl-2-imidazolidinone, ethyl acetate, propyl acetate, toluene, cyclopentylmethylether, tetrahydrofuran, 2-methyl tetrahydrofuran, dimethylsulfoxide, acetonitrile, propionitrile, dichloromethane and the like can be used. The solvent can be used two phase solvents with water or hydrous solvent, if necessary. Preferable examples of solvent include one or more solvents selected from the group consisting of polar solvent, toluene and dichloromethane.
Examples of polar solvents include one or more solvents selected from the group consisting of 1,2-dimethoxyethane, N, N-dimethylformamide, N,N-dimethylacetoamide, N-methyl pyrrolidone, 1,3-dimethyl-2-imidazolidinone, ethyl acetate, propyl acetate, cyclopentylmethylether, tetrahydrofuran, 2-methyl tetrahydrofuran, dimethylsulfoxide, acetonitrile, propionitrile, methylisobutylketone and the like. Preferable examples include one or more solvents selected from the group consisting of N, N-dimethylformamide, N,N-dimethylacetoamide, N-methyl pyrrolidone, 1,3-dimethyl-2-imidazolidinone, ethyl acetate, propyl acetate, cyclopentylmethylether, tetrahydrofuran, 2-methyl tetrahydrofuran, dimethylsulfoxide, acetonitrile, propionitrile and methylisobutylketone. Especially preferable examples include one or more solvents selected from the group consisting of tetrahydrofuran, N, N-dimethylacetoamide and methylisobutylketone.
When N, N-dimethylacetoamide and/or methylisobutylketone is used as a solvent, the compound of formula (IV) can be extracted with water. Since the Process 4 is high temperature reaction, when extracted with a low-boiling solvent such as ethyl acetate and the like, a low-boiling solvent has to be replaced by a high-boiling solvent. When N, N-dimethylacetoamide and/or methylisobutylketone is used as a solvent, it is not necessary to be replaced with solvent and to control the reaction temperature, it is very suitable for industrial use.
The temperature for such reaction is not limited, but usually can be about 0 to 100 °C and preferably about room temperature to 60 °C.
Reaction time is not limited, but usually can be conducted for 0.5 to 20 hours and preferably 1 to 10 hour(s).
After the completion of the reaction, the compound of formula (IV) is isolated and purified, and can be used in the next Process 4. The compound of formula (IV) filtered can be used in the next Process 4 without being isolated or purified. The compound of formula (IV) as a concentrate (For example, concentrated solution, slurry, frothy compound and the like) can be used in the Process 4 without being filtered.

The compound of formula (IV) obtained in the above process includes its salt or solvate thereof. The compound of formula (IV) is an important intermediate in the process for the preparation of the compound of formula (I) of the present invention. The compound of formula (I) can be prepared via the intermediate effectively. Using an explosive reagent can be avoided.
The compound of formula (IV): 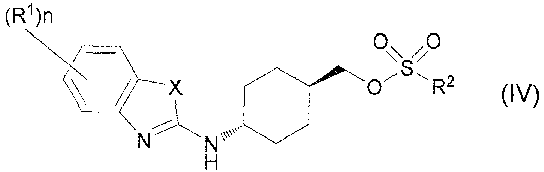
wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom, and
R² is C1-C6 alkyl; C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro,
its salt or solvate thereof is exemplified.
When R² is phenyl optionally substituted with one or more substituents selected from the group consisting of halogen, C1-C6 alkyl, C1-C6 alkoxy, C1-C6 haloalkoxy and nitro", phenyl can be optionally substituted with the substituent(s) at arbitrary position(s). Preferable example includes phenyl optionally substituted with the 1 to 3 substituent(s), especially preferable includes phenyl optionally substituted with the 1 to 2 substituents. When the phenyl is substituted with a number of substituents, the each substituent is can be same or different.
R¹ is preferably fluorine or chlorine.
n is preferably 0 or 1.
R² is preferably C1-C6 alkyl; C1-C6 haloalkyl; or phenyl optionally substituted with C1-C6 alkyl, and more preferably methyl, trifluoromethyl, phenyl and p-methylphenyl.

### Process 4

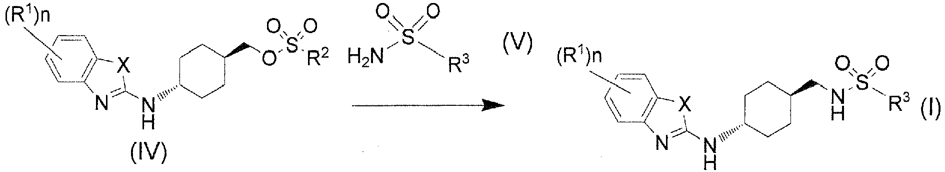

wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom,
R² is C1-C6 alkyl C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro; and
R³ is C1-C6 alkyl; C3-C8 cycloalkyl; or phenyl optionally substituted with one or more substituents selected from the group consisting of halogen, C1-C6 alkyl; C1-C6 alkoxy, C1-C6 haloalkyl and C1-C6 haloalkoxy.
The compound of formula (I), its salt or solvate thereof can be prepared to react the compound of formula (IV) with the compound of formula (V) in the presence of a base.
A base is not limited as long as it efficiently proceeds in the above process. Potassium tert-butoxide, sodium tert-butoxide, sodium methoxide, sodium ethoxide, sodium pentoxide, sodium phenoxide, sodium carbonate, potassium carbonate, calcium carbonate, cesium carbonate, magnesium carbonate, beryllium carbonate, sodium hydroxide, potassium hydroxide, calcium hydroxide, alkyllithium (e.g. n-butyllithium and the like), alkylmagnesium, strong base such as lithium amide (e.g. lithium diisopropylamide and the like), strong base such as hexamethyldisilazane (e.g. lithium hexamethyldisilazane, sodium hexamethyldisilazane, potassium hexamethyldisilazane and the like), alkyl magnesium halide (e.g. cyclohexyl magnesium bromide, isopropyl magnesium bromide, ethyl magnesium bromide, isopropyl magnesium chloride and the like) can be used. Preferable examples include alkali metal alkoxide or inorganic carbonate and the like.
"Alkali metal alkoxide" includes potassium tert-butoxide, sodium tert-butoxide, sodium methoxide, sodium ethoxide, sodium pentoxide, sodium phenoxide and the like. Furthermore, preferable examples include potassium tert-butoxide, sodium tert-butoxide, sodium methoxide, sodium ethoxide. Especially preferable example includes potassium tert-butoxide.
"Inorganic carbonate salt" includes sodium carbonate, potassium carbonate, calcium carbonate, cesium carbonate, magnesium carbonate, beryllium carbonate and the like. Preferable examples include sodium carbonate, potassium carbonate, calcium carbonate or cesium carbonate. Especially preferable example includes cesium carbonate.
Especially preferable examples of the base include potassium tert-butoxide or cesium carbonate in the above process.
The amount of the base can be 1mol to 10mol equivalent(s) to the Compound (IV). The amount of the base can be preferably 1mol to 8mol equivalent(s), especially preferably 1mol to 5mol equivalent(s) to the Compound (IV).
A solvent is not limited as long as it efficiently proceeds in the above process. One or more solvents selected from the group consisting of methanol, ethanol, isopropanol, n-propanol, tert-butanol, n-butanol, N, N-dimethylformamide, N,N-dimethylacetoamide, N-methyl pyrrolidone, 1,3-dimethyl-2-imidazolidinone, acetic acid, ethyl acetate, propyl acetate, toluene, cyclopentylmethylether, tetrahydrofuran, 2-methyl tetrahydrofuran, dimethylsulfoxide, acetonitrile, propionitrile, acetone, methylethylketone and the like can be used. The solvent can be used two phase solvents with water or hydrous solvent, if necessary. Preferable solvent includes polar solvent.
Examples of polar solvents include one or more solvents selected from the group consisting of methanol, ethanol, isopropanol, n-propanol, s-butanol, tert-butanol, n-butanol, N, N-dimethylformamide, N,N-dimethylacetoamide, N-methyl pyrrolidone, 1,3-dimethyl-2-imidazolidinone, acetic acid, ethyl acetate, propyl acetate, cyclopentylmethylether, tetrahydrofuran, 2-methyl tetrahydrofuran, dimethylsulfoxide, acetonitrile, propionitrile, acetone, methylethylketone, methylisobutylketone and the like. Preferable examples include one or more solvents selected from the group consisting of methanol, ethanol, isopropanol, n-propanol, tert-butanol, n-butanol, s-butanol, N, N-dimethylformamide, N,N-dimethylacetoamide, N-methyl pyrrolidone, 1,3-dimethyl-2-imidazolidinone, ethyl acetate, propyl acetate, tetrahydrofuran, 2-methyl tetrahydrofuran, dimethylsulfoxide, acetonitrile, propionitrile, acetone, methylethylketone, methylisobutylketone and the like. Moreover, preferable examples include one or more solvents selected from the group consisting of isopropanol, s-butanol, N, N-dimethylformamide, N,N-dimethylacetoamide, N-methyl pyrrolidone, methylisobutylketone and the like. Especially preferable examples include one or more solvents selected from the group consisting of isopropanol, s-butanol, N, N-dimethylformamide, N, N-dimethylacetoamide, methylisobutylketone and the like.
Using s-butanol as a solvent reduces generation of impurity, and improves the rate of removal of impurity in precipitation of the target. When toluene or cyclopentylmethylether is used as a solvent, toluene-aqueous sodium hydroxide, cyclopentylmethylether-aqueous sodium hydroxide and the like are used. Phase transfer catalyst (For example, tetrabutylammonium salt, octylmethylammonium salt, benzyldimethyloctadecyl ammonium salt and the like) may be added, if necessary.
The temperature for such reaction is not limited, but usually can be about 0 to 150 °C and preferably about room temperature to 100 °C.
Reaction time is not limited, but usually can be conducted for 0.5 to 20 hours and preferably 1 to 10 hour(s).
After the completion of the reaction, the reaction solution is concentrated and/or cooled to be precipitated solid. The precipitated solid is filtered to afford the compound of formula (I), its salt or solvate thereof. Solvent composition is adequately selected as described in the Example 6-2, it is not necessary to be condensed or purified with columns, it is very suitable for industrial use.

Examples are a compound of formula (I) synthesized by the above process: 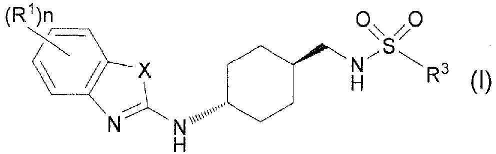
wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom,
R³ is C1-C6 alkyl; C3-C8 cycloalkyl; or phenyl optionally substituted with one or more substituents selected from the group consisting of halogen, C1-C6 alkyl, C1-C6 alkoxy, C1-C6 haloalkyl and C1-C6 haloalkoxy, and
its salt or solvate thereof.
When R³ is phenyl optionally substituted with one or more substituents selected from the group consisting of halogen, C1-C6 alkyl, C1-C6 alkoxy, C1-C6 haloalkyl and C1-C6 haloalkoxy, the phenyl can be substituted with the substituent(s) at arbitrary position(s). Preferable example includes phenyl optionally substituted with the 1 to 3 substituents, especially preferably includes phenyl optionally substituted with the 1 to 2 substituents. When the phenyl is substituted with a number of substituents, the each substituent is can be same or different.
R¹ is preferably fluorine or chlorine.
R³ is preferably methyl, ethyl, isopropyl, sec-butyl, tert-butyl, cyclopropyl, methoxyphenyl and fluorophenyl.
n is preferably 0 or 1.
Salts of a compound of formula (I) include hydrochloride salt of a compound of formula (I), sulfuric acid salt of a compound of formula (I) and the like.
Solvates of a compound of formula (I) include hydrate of a compound of formula (I), alcohol solvate of a compound of formula (I) and the like. Examples of solvates of a compound of formula (I) include monohydrate of a compound of formula (I), dihydrate of a compound of formula (I), monoalcohol solvate of a compound of formula (I), dialcohol solvate of a compound of formula (I) and the like. Preferable example includes hydrates containing less than two molecules.
A compound, its salt or solvate thereof of the formula (I) exhibits NPY Y5 receptor antagonistic activity and is very useful as a medicine especially for preventing or treating a disease associated with NPY Y5, e.g. feeding disorder, obesity, hyperorexia, sexual disorder, impaired fertility, depression, epileptic seizure, hypertension, cerebral hemorrhage, congestive heart failure or sleep disorders. Moreover, the antagonist is effective for preventing or treating the diseases in which obesity acts as a risk factor, for example, diabetes, hypertension, hyperlipemia, atherosclerosis and acute coronary syndrome.

### Example

### Example 1

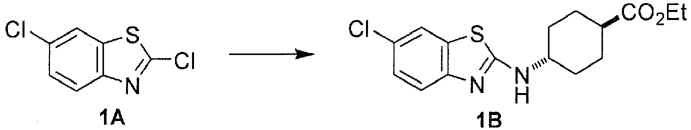

To N, N -dimethylformamide (26 mL) were added trans-4-amino-1-cyclohexanecarboxylic acid ethyl ester (5.19 g, 25 mmol) and triethylamine (12.5 mL, 90 mmol). The reaction suspension was stirred below 5 °C. To the reaction suspension was added N, N-dimethylformamide (10 mL) solution in Compound 1A (6.67 g, 32.5 mmol) dropwise, the reaction mixture was stirred at room temperature for 3 hours, and 60 °C for 3 hours. To the reaction solution were added ethyl acetate (62 mL) and 5%-citric acid solution (62 mL). The organic layer was extracted, and the water layer was repeatedly extracted with ethyl acetate (62 mL). The each of the organic layer was mixed. The mixed organic layer was washed with 5%-brine and dried over magnesium sulfate anhydrous. The solvent was removed under reduced pressure, and the obtained residue was purified using silica gel chromatography (chloroform-methanol 100:0→98:2(v/v)) to yield 2.53g of Compound 1B (yield 30 %) as a colorless solid.
¹H-NMR (CDCl₃) δ: 1.26 (t, J = 7.10 Hz, 3H), 1.28-1.34 (m, 2H), 1.58-1.68 (m, 2H), 2.02-2.14 (m, 2H), 2.24-2.34 (m, 2H), 3.52-3.66 (m, 1H), 4.14 (q, J = 7.10 Hz, 2H), 5.19 (s, 1H), 7.24 (dd, J = 8.62, 2.03 Hz, 1H), 7.41 (d, J = 8.62 Hz, 1H), 7.54 (d, J = 2.03 Hz, 1H)
MS: [M + H]⁺ m/z 339.0

### Example 2

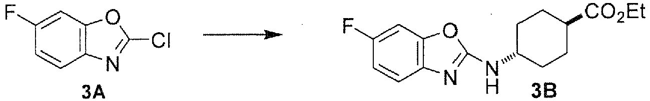

To N,N-dimethylformamide (10 mL) were added trans-4-amino-1-cyclohexanecarboxylic acid ethyl ester (2.08 g, 10 mmol) and triethylamine (5.0 mL, 36 mmol). The reaction solution was cooled below 5 °C and stirred. To the suspension, was added N,N-dimethylformamide (4mL) solution in Compound 3A(2.23 g, 13 mmol)(EP572893) dropwise, the reaction mixture was stirred at room temperature for 2 hours. To the reaction solution, were added ethyl acetate (25 mL) and 5%-citric acid solution (25 mL). The organic layer was extracted, the water layer was repeatedly extracted with ethyl acetate (25 mL). Each of the organic layer was mixed, and the mixed organic layer was washed with 5%-brine and dried over magnesium sulfate anhydrous. The solvent was removed under reduced pressure, and the residue was purified using silica gel chromatography (n-hexane - ethyl acetate 100:0→50:50(v/v)) to yield 3.04g of Compound 3B (yield 98 %) as a colorless solid.
¹H-NMR (CDCl₃) δ: 1.26 (t, J = 7.10 Hz, 3H), 1.27-1.38 (m, 2H), 1.58-1.68 (m, 2H), 2.04-2.13 (m, 2H), 2.23-2.34 (m, 2H), 3.63-3.79 (m, 1H), 4.14 (q, J = 7.10 Hz, 2H), 5.31 (s, 1H), 6.86-6.93 (m, 1H), 7.00 (dd, J = 8.24, 2.53 Hz, 1H), 7.23 (dd, J = 8.24, 4.82 Hz, 1H).
MS: [M + H]⁺ m/z 307.1

The following compounds were obtained in the similar manner above. Compound 2B (Yield 88%) 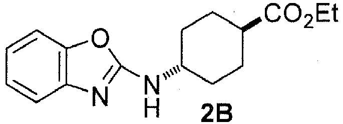 ¹H-NMR (CDCl₃) δ: 1.29 (t, J = 7.1 Hz, 3H), 1.31-1.40 (m, 2H), 1.59-1.74 (m, 2H), 2.06-2.15 (m, 2H), 2.26-2.35 (m, 3H), 3.73-3.82 (m, 1H), 4.16 (q, J = 7.1 Hz, 2H), 5.04 (d, J = 7.4 Hz, 1H), 7.05 (dd, J = 7.6, 7.6 Hz, 1H), 7.18 (dd, J = 7.6, 7.6 Hz, 1H), 7.26 (d, J = 7.6 Hz, 1H), 7.38 (d, J = 7.6 Hz, 1H).
MS: [M + H]⁺ m/z 289.0

### Compound 4B (Yield 78%)

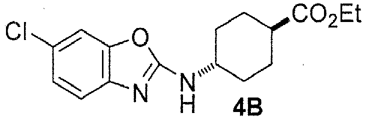

¹H-NMR (CDCl₃) δ: 1.26 (t, J = 7.10 Hz, 3H), 1.29-1.40 (m, 2H), 1.56-1.71 (m, 2H), 2.03-2.15 (m, 2H), 2.22-2.34 (m, 3H), 3.71 (m, 1H); 4.14 (q, J = 7.10 Hz, 2H), 5.07 (s, 1H), 6.98 (d, J = 8.11 Hz, 1H), 7.11-7.18 (m, 1H), 7.22-7.28 (m, 1H)
MS: [M + H]⁺ m/z 323.1

### Compound 5B (Yield 91%)

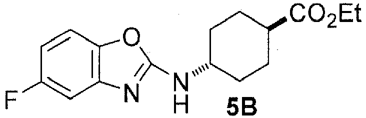

¹H-NMR (CDCl₃) δ: 1.26-1.40 (m, 2H), 1.29 (t, J = 7.2 Hz, 3H), 1.59-1.73 (m, 2H), 2.07-2.15 (m, 2H), 2.27-2.35 (m, 3H), 3.72-3.80 (m, 1H), 4.16 (q, J = 7.2 Hz, 2H), 4.92 (d, J = 8.1 Hz, 1H), 6.72-6.78 (m, 1H), 7.07 (dd, J = 8.8, 2.5 Hz, 1H), 7.15 (dd, J = 8.8, 4.4 Hz, 1H).
MS: [M + H]⁺ m/z 307.0

### Compound 6B (Yield 51%)

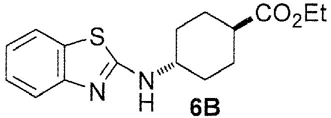

¹H-NMR (CDCl₃) δ: 1.25-1.37 (m, 2H), 1.28 (t, J = 7.2 Hz, 6H), 1.58-1.72 (m, 2H), 2.07-2.13 (m, 2H), 2.25-2.35 (m, 3H), 3.57-3.65 (m, 1H), 4.16 (q, J = 7.2 Hz, 2H), 5.40 (s, 1H), 7.10 (t, J = 7.6 Hz, 1H), 7.31 (t, J = 7.1 Hz, 1H), 7.53-7.62 (m, 2H).
MS: [M + H]⁺ m/z 305.2

### Compound 7B (Yield 29%)

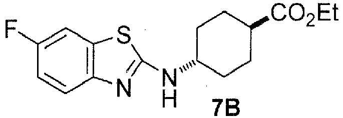

¹H-NMR (CDCl₃) δ: 1.24-1.36 (m, 2H), 1.29 (t, J = 7.1 Hz, 5H), 1.59-1.72 (m, 3H), 2.07-2.14 (m, 2H), 2.26-2.36 (m, 3H), 3.60-3.65 (m, 1H), 4.17 (q, J = 7.1 Hz, 2H), 5.11 (d, J = 6.9 Hz, 1H), 7.03 (td, J = 8.8, 2.7 Hz, 1H), 7.31 (dd, J = 8.8, 2.7 Hz, 1H), 7.46 (dd, J = 8.8, 4.8 Hz, 1H).
MS: [M + H]⁺ m/z 323.0

### Example 3

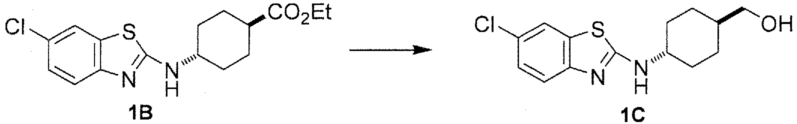

Compound 1B (2.03 g, 6.0 mmol) was dissolved in tetrahydrofuran - methanol (8.1mL-4.1mL), the reaction mixture was stirred and heated at 60 °C, and to the reaction mixture was added LiBH₄ (2.0M-tetrahydrofuran solution, 6 mL, 12 mmol) dropwise over 2 hours. This reaction solution was stirred at 60°C for 2 hours, and were added methanol (2.1 mL) and LiBH₄ (2.0M-tetrahydrofuran solution, 3 mL, 6 mmol) dropwise. The reaction mixture was stirred and heated at 60 °C for 1 hour and 30 minutes. The reaction solution was cooled to 5 °C. 2N-hydrogen chloride, 2N- sodium hydroxide (10mL), 5%- sodium hydrogen carbonate (12mL) was added to the reaction solution. The reaction mixture was extracted with ethyl acetate. The water layer was extracted with ethyl acetate (24 mL). The each of organic layer was mixed. The mixed organic layer was washed with brine (12 mL), dried over anhydrous magnesium sulfate. The solvent was removed under reduced pressure, and the residue was purified using silica gel chromatography (ethyl acetate-hexane 0:100→ 50:50(v/v)) to yield 0.97g of a compound 1C (yield 54 %) as a colorless solid.
¹H-NMR (DMSO-d₆) δ: 0.93-1.07 (m, 2H), 1.14-1.28 (m, 2H), 1.28-1.41 (m, 1H), 1.78 (d, J = 11.66 Hz, 2H), 2.06 (d, J = 10.14 Hz, 2H), 3.23 (t, J = 5.58 Hz, 2H), 3.54-5.57 (m, 1H), 4.44 (t, J = 5.32 Hz, 1H), 7.21 (dd, J = 8.62, 2.03 Hz, 1H), 7.34 (d, J = 8.62 Hz, 1H), 7.76 (d, J = 2.03 Hz, 1H), 8.05 (d, J = 7.60 Hz, 1H).
MS: [M + H]⁺ m/z 297.1

### Example 4-1

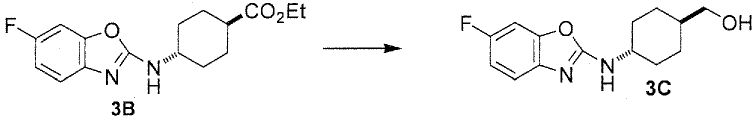

The compound 3B (1.23 g, 4.0 mmol) was dissolved in tetrahydrofuran - methanol (6.0 mL-5.0 mL). The reaction mixture was stirred and heated at 70 °C, and to the reaction mixture was added LiBH4 (2.0M-tetrahydrofuran solution, 4.0 mL, 8.0 mmol) dropwise for 2 hours. This reaction solution was stirred at 70 °C for 1 hour. To the reaction solution, were added methanol (1.2 mL), tetrahydrofuran (1.2 mL) and LiBH4 (2.0M-tetrahydrofuran solution, 4.0 mL, 8.0 mmol) dropwise over 2 hours. The reaction mixture was stirred and heated at 70 °C for 1 hour. Then the reaction mixture was cooled to 5 °C. 2N-hydrogen chloride (32 mL), 2N- sodium hydroxide (24 mL) and 5%-sodium hydrogen carbonate (12mL) was added to the reaction mixture. The reaction mixture was extracted with ethyl acetate. The water layer was extracted with ethyl acetate (15 mL). The each of organic layer was mixed. The mixed organic layer was washed with brine (7.5 mL), dried over anhydrous sodium sulfate. The solvent was removed under reduced pressure, and the residue was washed with n-hexane and isopropylether to yield 0.95g of a compound 3C (yield 91 %) as a colorless light brown solid.
1H-NMR (DMSO-d6) δ: 0.91-1.06 (m, 2H), 1.19-1.40 (m, 3H), 1.79 (d, J = 11.66 Hz, 2H), 2.00-2.08 (m, 2H), 3.20-3.26 (m, 2H), 3.43-3.52 (m, 1H), 4.41-4.49 (m, 1H), 6.92-6.97 (m, 1H), 7.19 (dd, J = 8.49, 4.82 Hz, 1H), 7.32 (dd, J = 8.49, 2.53 Hz, 1H), 7.87 (d, J = 8.11 Hz, 1H).
MS: [M + H]+ m/z 265.0

Compound 3C is also able to be obtained in the following manner.

### Example 4-2

Compound 3B (10.00g, 32.64mmol) was dissolved in methanol (30 mL). To this reaction solution, was added 4.96g of calcium chloride (95%), and the reaction solution was heated at 50±5 °C. NaBH₄ (98.5%)(2.13g, 1.7eq) was scaled, added into another reaction vessel, then N, N-dimethylacetamide solution (1.7mL) was added to the vessel. To the solution in Compound 3B, was added the NaBH₄ solution dropwise at 50±5 °C for 4 hours. The reaction solution was reacted at 50±5 °C. After the completion of the reaction, the reaction solution was cooled to 25±5 °C. After the reaction solution had been left under the nitrogen atmosphere, then the slurry was stirred and acetone (2.5mL) was added to the slurry. After stirring for 2 hours, propionic acid (99.0%) (4.40g, 1.8eq) was added to the slurry. The reaction solution was stirred for 1 hour, then tap water was added dropwise for 1 hour. After the completion of the reaction, the solution was crystallized at 25±5 °C for more than 1. hour, and filtered. The obtained undried crystal was washed with 20% of methanol-water (50mL) to yield Compound 3C as wet crystal.
Wet crystal of Compound 3C was dried under reduced pressure for 5 hours to yield 8.03g of Compound 3C as a dried crystal.

The following compounds were obtained in the similar manner above.

### Compound 2C (Yield 73%)

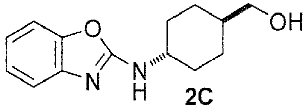

¹H-NMR (DMSO-d₆) 8: 0.93-1.06 (m, 2H), 1.19-1.44 (m, 3H), 1.79 (dd, J = 12.17 Hz, 2H), 2.04 (dd, J = 10.14 Hz, 2H), 3.24 (t, J = 5.58 Hz, 1H), 3.44-3.50 (m, 1H), 4.45 (t, J = 5.32 Hz, 1H), 6.96 (t, J = 7.60 Hz, 1H), 7.10 (t, J = 7.60 Hz, 1H), 7.23 (d, J = 8.11 Hz, 1H), 7.31 (d, J = 8.11 Hz, 1H), 7.82 (d, J = 8.11 Hz, 1H)
MS: [M + H]⁺ m/z 247.1

### Compound 4C (Yield 46%)

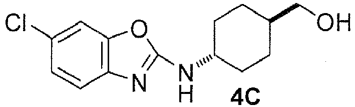

¹H-NMR (DMSO-d₆) δ: 0.91-1.05 (m, 2H), 1.20-1.39 (m, 3H), 1.79 (d, J = 12.17 Hz, 2H), 2.03 (d, J = 9.63 Hz, 2H), 3.23 (t, J = 5.58 Hz, 2H), 3.40-3.53 (m, 1H), 4.38-4.44 (m, 1H), 7.11-7.16 (m, 1H), 7.21 (d, J = 8.62 Hz, 1H), 7.48 (d, J = 2.03 Hz, 1H), 8.01 (d, J = 7.60 Hz, 1H).
MS: [M + H]⁺ m/z 281.0

### Compound 5C (Yield 97%)

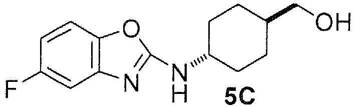

¹H-NMR (DMSO-d₆) δ: 0.95-1.06 (m, 2H), 1.22-1.36 (m, 2H), 1.78-1.84 (m, 2H), 2.02-2.08 (m, 2H), 3.25 (dd, J = 5.7, 5.3 Hz, 2H), 3.45-3.55 (m, 1H), 4.43 (t, J = 5.3 Hz, 1H), 6.77 (ddd, J = 10.6, 8.5, 2.2 Hz, 1H), 7.08 (dd, J = 9.3, 2.2 Hz, 1H), 7.33 (dd, J = 8.5, 4.5 Hz, 1H), 8.04 (d, J = 7.9 Hz, 1H).
MS: [M + H]+ m/z 265.1

### Compound 6C (Yield 61%)

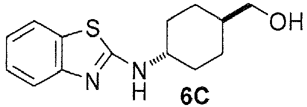

¹H-NMR (DMSO-d₆) δ: 0.96-1.41 (m, 9H), 1.78-1.84 (m, 2H), 2.07-2.13 (m, 2H), 3.26 (dd, J = 5.8, 5.4 Hz, 1H), 3.60-3.68 (m, 1H), 4.42 (dd, J = 5.4, 5.3 Hz, 1H), 7.01 (t, J = 7.8 Hz, 1H), 7.22 (t, J = 7.8 Hz, 1H), 7.39 (d, J = 7.8 Hz, 1H), 7.66 (d, J = 7.8 Hz, 1H), 7.93 (d, J = 7.4 Hz, 1H).
MS: [M + H]+ m/z 262.9

### Compound 7C (Yield 30%)

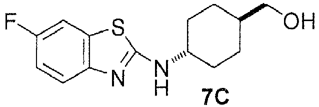

¹H-NMR (DMSO-d₆) δ: 0.93-1.05 (m, 2H), 1.15-1.34 (m, 3H), 1.74-1.81 (m, 2H), 2.04-2.09 (m, 2H), 3.23 (dd, J = 5.8, 5.3 Hz, 2H), 3.54-3.64 (m, 1H), 4.40 (t, J = 5.3 Hz, 1H), 7.02 (td, J = 9.0, 2.7 Hz, 1H), 7.34 (dd, J = 8.8, 4.9 Hz, 1H), 7.56 (dd, J = 9.0, 2.6 Hz, 1H), 7.90 (d, J = 7.2 Hz, 1H).
MS: [M + H]+ m/z 281.0

### Example 5

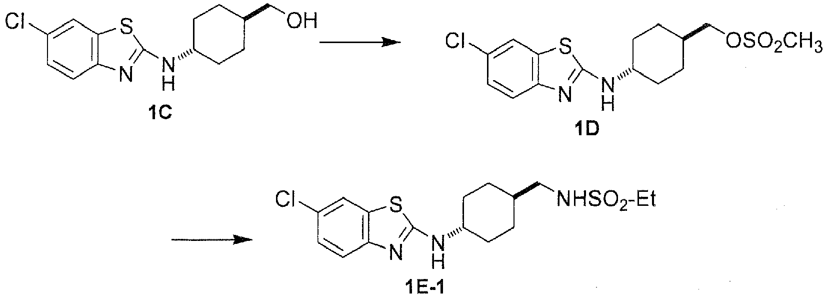

Compound 1C (888 mg, 3.0 mmol) was dissolved in tetrahydrofuran (6.0 mL), to the reaction solution were added triethylamine (0.50 mL, 3.6 mmol) and methanesulfonyl chloride(0.26 mL, 3.3 mmol). The reaction mixture was stirred at room temperature for 2 hours. The reaction mixture was poured into iced water. The reaction mixture was extracted with ethyl acetate (20 mL x 2). The organic layer was washed with iced water (20 mL x 2), dried over anhydrous sodium sulfate. The solvent was removed under reduced pressure to obtain 1.146 g of Compound 1E as a frothy compound.
¹H-NMR (DMSO-d₆) δ: 8.21 (1H, d, J = 6.6 Hz), 7.78 (1H, d, J = 2.5 Hz), 7.35 (1H, d, J = 8.6 Hz), 7.23 (1H, dd, J = 8.6, 2.5 Hz), 4.05 (3H, d, J = 5.6 Hz), 3.70-3.58 (1H, m), 3.15 (3H, s), 2.14-2.05 (2H, m), 1.80-1.64 (3H, m), 1.36-1.10 (5H, m).
286mg of the above Compound 1E was dissolved in dimethylformamide (2.7 mL). To the solution were added ethanesulfonyl chloride (146 mg, 1.3 mmol) and potassium t-butoxide (95 %, 133 mg, 1.12 mmol), the reaction solution was stirred at 85 °C for 3 hours. The reaction solution was poured into iced water. The reaction solution was extracted with ethyl acetate (20 mL x 2). The organic layer was washed with iced water (20 mL x 2), dried over anhydrous sodium sulfate. The solvent was removed under reduced pressure, and the residue was purified using silica gel chromatography (toluene-ethyl acetate 1:1(v/v)) to yield 139mg of a compound 1E-1 (yield 43 %) as a white solid.
Anal. Calcd for C_{1 6} H_{2 2} ClN₃ O₂ S₂ : C 49.54, H 5.72, Cl 9.14, N 10.83, S 16.53
Found: C 49.55, H 5.67, Cl 9.18, N 10.78, S 16.39
¹H-NMR (DMSO-d₆) δ: 0.97-1.05 (m, 2H), 1.18-1.24 (m, 2H), 1.16 (t, 3H, J = 7.5 Hz), 1.34-1.41 (m, 1H), 1.77-1.81 (m, 2H), 2.02-2.08 (m, 2H), 2.76 (t, 2H, J = 6.0 Hz), 2.96 (q, 2H, J = 7.5 Hz), 3.55-3.64 (m, 1H), 7.00 (t, 1H, J = 7.8 Hz), 7.18 (dd, 1H, J = 8.4, 1.8 Hz), 7.32 (dd, 1H, J = 8.4, 0.6 Hz), 7.74 (d, 1H, J = 1.8 Hz), 8.04 (d, 1H, J = 7.8 Hz).

### Example 6-1

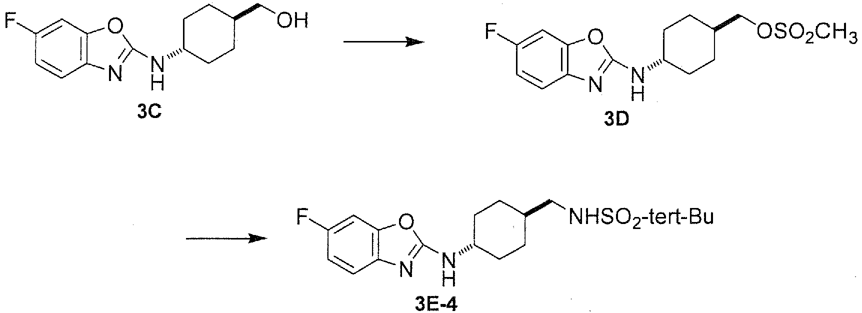

Compound 3C (794 mg, 3.0 mmol) was dissolved in dimethylacetamide (6 mL), to the reaction solution were added triethylamine (1.00 mL, 7.2 mmol) and methanesulfonyl chloride (0.47 mL, 6.0 mmol) with ice-cooling, then the reaction mixture was stirred at room temperature for 2 hours. The reaction mixture was poured into iced water. The reaction mixture was extracted with ethyl acetate (20 mL x 2). The organic layer was washed with iced water (20 mL x 2), dried over anhydrous sodium sulfate. The solvent was removed under reduced pressure to yield 1.215 g of crude Compound 3D.
¹ H-NMR (300MHz, DMSO-d₆) δ: 1.05-1.22 (m, 2H), 1.22-1.40 (m, 2H), 1.60-1.76 (m, 1H), 1.76-1.86 (m, 2H), 2.02-2.12 (m, 2H), 3.18 (s, 3H), 3.38-3.56 (m, 1H), 4.05 (d, 2H, J = 6.3 Hz), 6.92-7.00 (m, 1H), 7.20 (dd, 1H, J =8,4, 5.1 Hz), 7.35 (dd, 1H, J = 8.7, 2.1 Hz), 8.00 (d, 1H, J = 7.5 Hz).
To mixed solution in dimethylacetamide (2 mL) and ethyl acetate (0.5 mL) in tert-butyl sulfonamide (186 mg, 1.35 mmol) and cesium carbonate (326 mg, 1.00 mmol), was added 304mg of the above crude Compound 3D. The reaction mixture was stirred at 80 °C for about 15 hours. The reaction mixture was poured into iced water (25 mL). The reaction mixture was extracted with ethyl acetate (20 mL x 2). The organic layer was washed with iced water (20 mL x 2), dried over anhydrous sodium sulfate. The solvent was removed under reduced pressure, and the residue was purified using silica gel chromatography (chloroform-methanol 10:0 → 10:1(v/v)) to yield 201.6 mg of a compound 3E-4 (yield 70 %) as a white solid.
Anal. Calcd for C₁₈ H₂₆ FN₃ O₃ S : C 56.38, H 6.83, F 4.95, N 10.96, S 8.36
Found : C 56.35, H 6.92, F 5.15, N 10.82, S 8.57
¹H-NMR (DMSO-d₆) δ: 0.91-1.08 (m, 2H), 1.16-1.46 (m, 3H), 1.27 (s, 9H), 1.77-1.87 (m, 2H), 1.98-2.10 (m, 2H), 2.89 (t, 2H, J = 6.0 Hz), 3.38-3.54 (m, 1H), 6.88 (t, 1H, J = 5.7 Hz), 6.90-7.00 (m, 1H), 7.19 (dd, 1H, J = 8.7, 5.1 Hz), 7.34 (dd, 1H, J = 8.7, 2.7 Hz), 7.88 (d, 1H, J = 7.5 Hz).

Compound 3D and 3E-4 are also able to be obtained in the following manner.

### Example 6-2

To Compound 3C (10.0 g, 37.8 mmol) was added N, N-dimethylacetamide (30 mL). The reaction solution was heated to 35±5 °C, were then added methyl isobutyl ketone (30 mL) and triethylamine (1.7eq, 6.5g) to the reaction mixture. The reaction solution was cooled to 0±5 °C, methanesulfonyl chloride (1.2eq, 5.2 g) was added dropwise for more than 60 minutes to the reaction solution. The reaction solution was reacted at the same temperature for 1 hour. Then to the reaction mixture was added water (20 mL) dropwise for about 30 minutes, the reaction mixture was extracted. The organic layer was washed with water (10 mL). The water layer was back extracted with methyl isobutyl ketone (30 mL). The extracted solution including the obtained Compound 3D was condensed under reduced pressure to 3.6W (36 g).
To the n-butanol slurry (30 mL) in t-butyl sulfonamide (2.0eq, 10.4 g) and cesium carbonate (1.5eq, 18.5 g), was added the concentrated solution including Compound 3D dropwise at 90±5 °C for 4 hours. The reaction solution was stirred at same temperature for 3 hours, then cooled to 80 °C To the reaction solution was added water (10 mL), the water layer was withdrawn from the reaction solution at 60±10 °C. To the organic layer, was added water (30 mL) dropwise at the same temperature for 30 minutes, the mixture was cooled to 0±5 °C. The precipitated solids were collected with filtration, the obtained solids were washed with 50% 2-propanol water (60 mL) to give 12.4g (83.2%) of Compound 3E-4.

The following compounds were obtained to be described in yield by the same process above.

### Compound 1E-2 (Yield 61%)

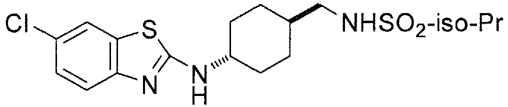

¹H-NMR (DMSO-d₆) δ: 8.05 (1H, d, J = 7.0 Hz), 7.76 (1H, d, J = 2.0 Hz), 7.34 (1H, d, J = 8.6 Hz), 7.27-7.13 (1H, m), 6.97 (1H, t, J = 6.1 Hz), 3.64-3.60 (1H, m), 3.15-3.10 (1H, m), 2.81 (2H, t, J = 11.7 Hz), 2.08-2.05 (2H, m), 1.83-1.80 (2H, m), 1.43-1.31 (1H, m), 1.25-1.15 (2H, m), 1.21 (6H, d, J = 6.6 Hz), 1.05-0.97 (2H, m), 1.06-0.96(1H, m).

### Compound 1E-3 (Yield 74%)

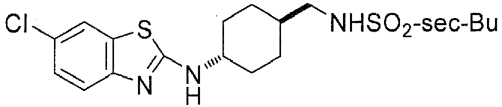

¹H-NMR (DMSO-d₆) δ: 8.02 (1H, d, J = 6.6 Hz), 7.76 (1H, d, J = 2.5 Hz), 7.34 (1H, d, J = 8.6 Hz), 6.99 (1H, t, J = 6.1 Hz), 6.65 (1H, s), 3.64-3.60 (1H, m), 2.96-2.87 (1H, m), 2.83-2.73 (2H, m), 2.09-2.05 (2H,m), 1.98-1.80 (2H, m), 1.45-1.32 (2H, m), 1.30-1.11 (5H, m), 1.05-0.96 (3H,m), 0.95 (3H, t, J = 7.6Hz).

### Compound 1E-4(Yield 49%)

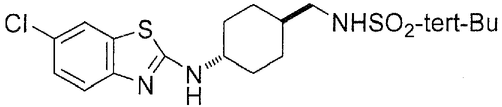

¹H-NMR (DMSO-d₆) δ: 1.01 (dd, 2H, J = 24.6, 10.2 Hz), 1.21 (dd, 2H, J = 24.6, 10.2 Hz), 1.27 (s, 9H), 1.34-1.40 (m, 1H), 1.82 (d, 2H, J = 11.2 Hz), 2.08 (d, 2H, J = 11.2 Hz), 2.89 (t, 2H, J = 6.2 Hz), 3.59-3.65 (m, 1H), 6.87 (t, 1H, J = 5.8 Hz), 7.21 (dd, 1H, J = 8.6, 2.4 Hz), 7.34 (d, 1H, J = 8.6 Hz), 7.77 (d, 1H, J = 1.8 Hz), 8.06 (d, 1H, J = 7.6 Hz).

### Compound 2D

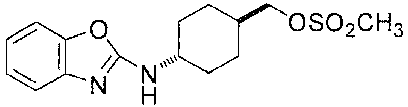

¹H-NMR (DMSO-d₆) δ: 1.07-1.22 (m, 2H), 1.24-1.39 (m, 2H), 1.62-1.75 (m, 1H), 1.81 (d, J = 12.17 Hz, 2H), 2.07 (d, J = 10.14 Hz, 2H), 3.18 (s, 3H), 3.45-3.57 (m, 1H), 4.05 (t, J = 6.59 Hz, 1H), 6.96 (t, J = 7.60 Hz, 1H), 7.10 (t, J = 7.60 Hz, 1H), 7.23 (d, J =7.60 Hz, 1H), 7.31 (d, J = 8.11 Hz, 1H), 7.89 (d, J = 7.60 Hz, 1H).

### Compound 2E-1 (Yield 61%)

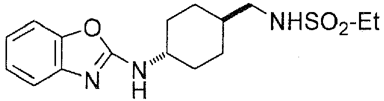

¹H-NMR (DMSO-d₆) δ: 0.933-1.10 (m, 2H), 1.12-1.30 (m, 2H), 1.19 (t, 3H, J = 7.2 Hz), 1.39 (m, 1H), 1.77-1.87 (m, 2H), 1.99-2.12 (m, 2H), 2.78 (t, 2H, J = 6.6 Hz), 2.98 (q, 2H, J = 7.2 Hz), 3.40-3.58 (m, 1H), 6.95 (t, 1H, J = 7.8 Hz), 7.02 (t, 1H, J = 6.0 Hz), 7.09 (t, 1H, J = 7.8 Hz), 7.22 (d, 1H, J = 7.5 Hz), 7.31 (d, 1H, J = 8.1 Hz), 7.83 (d, 1H, J = 7.8 Hz).

### Compound 2E-2 (Yield 45%)

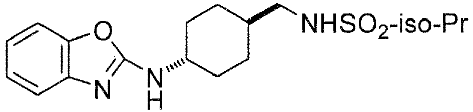

¹H-NMR (DMSO-d₆) δ: 0.95-1.16 (m, 2H), 1.18-1.44 (m, 3H), 1.21 (d, 6H, J = 6.6 Hz), 1.78-1.86 (m, 2H), 2.02-2.12 (m, 2H), 2.78-2.84 (m, 2H), 3.10-3.20 (m, 1H), 3.40-3.58 (m, 1H), 6.95 (t, 1H, J = 7.8 Hz), 7.01 (brs, 1H), 7.09 (t, 1H, J = 6.9 Hz), 7.22 (d, 1H, J = 6.6 Hz), 7.31 (d, 1H, J = 7.8 Hz), 7.83 (d, 1H, J = 7.8 Hz).

### Compound 2E-3 (Yield 79%)

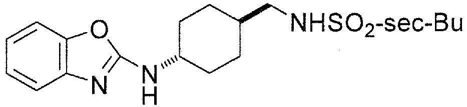

¹H-NMR (DMSO-d₆) δ: 0.96 (t, J = 7.6 Hz, 3H), 0.94-1.07 (m, 2H), 1.22 (t, J = 6.34 Hz, 3H, 1.22-1.33 (m, 2H), 1.33-1.45 (m, 2H), 1.83 (d, J = 11.15 Hz, 2H), 1.84-1.95 (m, 1H), 2.04 (d, J = 13.69 Hz, 2H), 2.80 (t, J = 6.34 Hz, 2H), 2.86-2.97 (m, 1H), 3.43-3.55 (m, 1H), 6.95 (t, J = 8.36 Hz, 1H), 6.99 (t, J = 7.60 Hz, 1H), 7.09 (t, J = 7.60 Hz, 1H), 7.22 (d, J =7.10 Hz, 1H), 7.31 (d, J = 7.60 Hz, 1H), 7.82 (d, J = 7.60 Hz, 1H).

### Compound 2E-4 (Yield 72%)

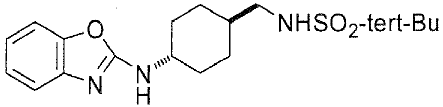

¹H-NMR (DMSO-d₆) δ: 0.90-1.08 (m, 2H), 1.12-1.40 (m, 3H), 1.25 (s, 9H), 1.76-1.86 (m, 2H), 1.98-2.10 (m, 2H), 2.87 (d, 2H, J = 6.3 Hz), 3.40-3.56 (m, 1H), 6.85 (brs, 1H), 6.93 (t, 1H, J = 7.5 Hz), 7.07 (t, 1H, J = 7.5 Hz), 7.20 (d, 1H, J = 7.5 Hz), 7.29 (d, 1H, J = 7.8 Hz), 7.79 (brs, 1H).

### Compound 3E-1 (Yield 46%)

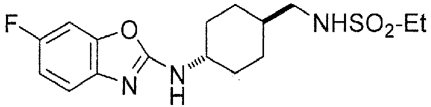

¹H-NMR (DMSO-d₆) δ: 0.94-1.08 (m, 2H), 1.16-1.33 (m, 2H), 1.19 (t, 3H, J = 7.2 Hz), 1.33-1.45 (m, 1H), 1.77-1.86 (m, 2H), 2.00-2.08 (m, 2H), 2.74-2.82 (m, 2H), 2.98 (q, 2H, J = 7.2 Hz), 3.38-3.54 (m, 1H), 6.90-7.00 (m, 1H), 7.02 (t, 1H, J = 4.5 Hz), 7.19 (dd, 1H, J = 8.4, 5.1 Hz), 7.33 (dd, 1H, J = 8.4, 2.7 Hz), 7.88 (d, 1H, J = 7.8 Hz).

### Compound 3E-2 (Yield 83%)

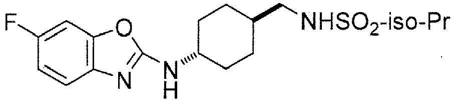

¹H-NMR (DMSO-d₆) δ: 0.92-1.08 (m, 2H), 1.20-1.34 (m, 2H), 1.22 (d, 6H, J = 6.9 Hz), 1.38 (m, 1H), 1.78-1.86 (m, 2H), 2.00-2.14 (m, 2H), 2.81 (t, 2H, J = 6.3 Hz), 3.10-3.21 (m, 1H), 3.38-3.54 (m, 1H), 6.90-7.00 (m, 2H), 7.19 (dd, 1H, J = 8.4, 4.8 Hz), 7.33 (dd, 1H, J = 8.4, 2.4 Hz), 7.86 (d, 1H, J = 7.8 Hz).

### Compound 3E-3 (Yield 81%)

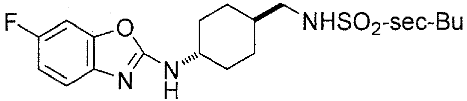

¹H-NMR (DMSO-d₆) δ: 0.95 (t, 3H, J = 7.5 Hz), 0.92-1.08 (m, 2H), 1.21 (d, 3H, J = 6.9 Hz), 1.20-1.47 (m, 4H), 1.77-1.96 (m, 3H), 1.98-2.08 (m, 2H), 2.79 (t, 2H, J = 6.3 Hz), 2.85-2.98 (m, 1H), 3.38-3.56 (m, 1H), 6.90-6.98 (m, 1H), 7.00 (t, 1H, J = 6.0 Hz), 7.19 (dd, 1H, J = 8.7, 5.1 Hz), 7.33 (dd, 1H, J = 8.7, 2.7 Hz), 7.88 (d, 1H, J = 7.8 Hz).

### Compound 4D

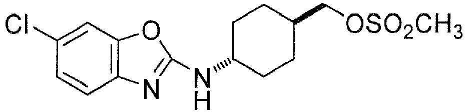

¹H-NMR (DMSO-d₆) δ: 1.04-1.23 (m, 2H), 1.23-1.39 (m, 2H), 1.55-1.74 (m, 1H), 1.81 (d, J = 12.67 Hz, 2H), 2.06 (d, J = 12.17 Hz, 2H), 3.17 (s, 3H), 3.41-3.57 (m, 1H), 4.05 (d, J = 6.08 Hz, 2H), 7.14 (dd, J = 8.11, 2.03 Hz, 1H), 7.21 (d, J = 8.11 Hz, 1H), 7.49 (d, J = 2.03 Hz, 1H), 8.05 (d, J = 7.60 Hz, 1H).

### Compound 4E-1 (Yield 63%)

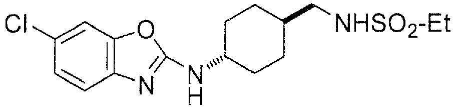

¹H-NMR (DMSO-d₆)δ: 0.92-1.08 (m, 2H), 1.15-1.33 (m, 2H), 1.19 (t, 3H, J = 7.2 Hz), 1.33-1.42 (m, 1H), 1.76-1.86 (m, 2H), 1.98-2.08 (m, 2H), 2.76-2.82 (m, 2H), 2.97 (q, 2H, J = 7.2 Hz), 3.40-3.58 (m, 1H), 7.01 (t, 1H, J = 6.0 Hz), 7.13 (d, 1H, J = 8.4 Hz), 7.20 (d, 1H, J = 8.4 Hz), 7.49 (s, 1H), 8.01 (d, 1H, J = 7.6 Hz).

### Compound 4E-2 (Yield 66%)

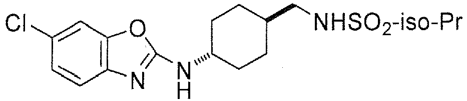

¹H-NMR (DMSO-d₆) δ: 0.91 -1.08 (m, 2H), 1.17-1.33 (m, 8H), 1.33-1.44 (m, 1H), 1.82 (d, J = 12.17 Hz, 2H), 2.03 (d, J = 9.63 Hz, 2H), 2.80 (t, J = 6.09 Hz, 2H), 3.11-3.18 (m, 1H), 3.40-3.55 (m, 1H), 6.97 (t, J = 6.09 Hz, 1H), 7.14 (dd, J = 8.49, 2.03 Hz, 1H), 7.21 (d, J = 8.49 Hz, 1H), 7.69 (d, J = 2.03 Hz, 1H), 8.01 (d, J = 7.60 Hz, 1H).

### Compound 4E-3 (Yield 74%)

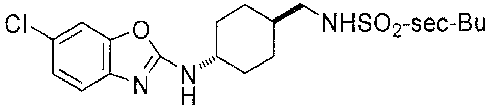

¹H-NMR (DMSO-d₆) δ: 9.95 (t, J = 7.35 Hz, 3H), 0.93 -1.05 (m, 2H), 1.21 (t, J = 6.59 Hz, 3H), 1.21-1.33 (m, 3H), 1.33-1.44 (m, 2H), 1.82 (d, J = 11.66 Hz, 2H), 2.03 (d, J = 10.14 Hz, 2H), 2.79 (t, J = 6.09 Hz, 2H), 2.97-87 (m, 1H), 3.42-3.54 (m, 1H), 6.99 (t, J = 6.09 Hz, 1H), 7.13 (dd, J = 8.62, 2.03 Hz, 1H), 7.21 (d, J = 8.62 Hz, 1H), 7.49 (d, J = 2.03 Hz, 1H), 8.01 (d, J = 7.60 Hz, 1H).

### Compound 4E-4 (Yield 78%)

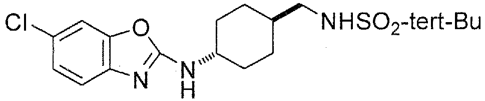

¹H-NMR (CDCl₃) δ: 1.08-1.26 (m, 2H), 1.36-1.60 (m, 3H), 1.40 (s, 9H), 1.92-2.02 (m, 2H), 2.22-2.32 (m, 2H), 3.08 (t, 2H, J = 6.6 Hz), 3.68-3.80 (m, 1H), 4.03 (t, 1H, J = 6.0 Hz), 7.06 (brs, 1H), 7.20-7.36(m, 3H).

### Compound 5D

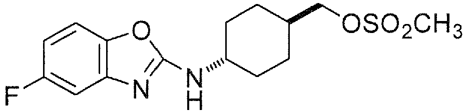

¹H-NMR (DMSO-d₆) δ: 8.69 (1H, d, J = 6.6 Hz), 7.40 (1H, dd, J = 9.1, 4.3 Hz), 7.14 (1H, dd, J = 9.1, 2.2 Hz), 6.84 (1H, td, J = 9.1, 2.2 Hz), 4.05 (2H, d, J = 6.6 Hz), 3.56-3.52 (1H, m), 3.18 (3H, s), 2.08-2.04 (2H, m), 1.84-1.80 (2H, m), 1.74-1.64 (1H, m), 1.39-1.29 (2H, m), 1.19-1.09 (2H, m).

### Compound 5E-1 (Yield 49%)

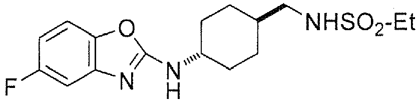

¹H-NMR (DMSO-d₆) δ: 8.02 (1H, d, J = 7.6 Hz), 7.31 (1H, dd, J = 8.6, 4.6 Hz), 7.07-7.00 (2H, m), 6.78-6.72 (1H, m), 3.54-5.42 (1H, m), 2.98 (2H, q, J = 8.6 Hz), 2.77 (2H, t, J = 6.1 Hz), 2.04-2.01 (2H, m), 1.84-1.81 (2H, m), 1.40-1.22 (3H, m), 1.19 (3H, t, J = 8.6Hz), 1.04-0.96 (2H, m).

### Compound 5E-2 (Yield 45%)

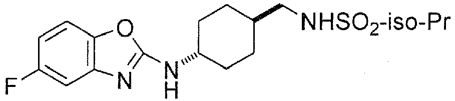

¹H-NMR (DMSO-d₆) δ: 0.92-1.08 (m, 2H), 1.20-1.34 (m, 2H), 1.22 (d, 6H, J = 6.8 Hz), 1.38 (m, 1H), 1.78-1.86 (m, 2H), 1.99-2.14 (m, 2H), 2.80 (t, 2H, J = 6.4 Hz), 3.10-3.20 (m, 1H), 3.42-3.54 (m, 1H), 6.74 (td, 1H, J = 8.8, 2.8 Hz), 6.98 (t, 1H, J = 5.6 Hz), 7.06 (dd, 1H, J = 9.6, 2.8 Hz), 7.30 (dd, 1H, J = 8.0, 4.4 Hz), 8.02 (d, 1H, J = 8.0 Hz).

### Compound 5E-3 (Yield 72%)

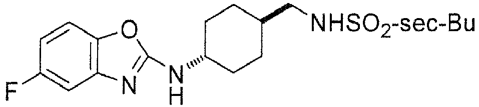

¹H-NMR (DMSO-d₆) δ: 0.95 (t, 3H, J = 7.8 Hz), 0.92-1.07 (m, 2H), 1.21 (d, 3H, J = 6.9 Hz), 1.20-1.47 (m, 4H), 1.77-1.96 (m, 3H), 1.98-2.08 (m, 2H), 2.79 (t, 2H, J = 6.3 Hz), 2.85-2.98 (m, 1H), 3.42-3.57 (m, 1H), 6.71-6.80 (m, 1H), 7.00 (t, 1H, J = 5.7 Hz), 7.06 (dd, 1H, J = 9.3, 2.7 Hz), 7.31 (dd, 1H, J = 8.7, 4.5 Hz), 8.02 (d, 1H, J = 7.8 Hz).

### Compound 5E-4 (Yield 51%)

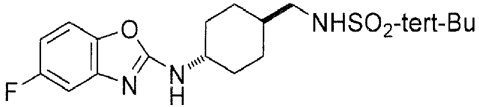

¹H-NMR (DMSO-d₆) δ: 0.92-1.08 (m, 2H), 1.19-1.34 (m, 2H), 1.27 (s, 9H), 1.38 (m, 1H), 1.78-1.86 (m, 2H), 1.99-2.14 (m, 2H), 2.88 (t, 2H, J = 6.3 Hz), 3.40-3.56 (m, 1H), 6.75 (td, 1H, J = 8.7, 2.7 Hz), 6.87 (t, 1H, J = 6.0 Hz), 7.06 (dd, 1H, J = 9.3, 2.7 Hz), 7.31 (dd, 1H, J = 8.7, 4.5 Hz), 8.02 (d, 1H, J = 7.8 Hz).

### Compound 6D

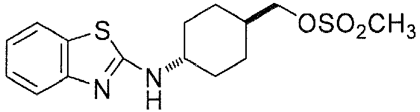

¹H-NMR (DMSO-d₆) δ: 1.09-1.21 (m, 2H), 1.21-1.32 (m, 2H), 1.62-1.75 (m, 1H), 1.81 (d, J = 12.17 Hz, 2H), 2.10 (d, J = 9.63 Hz, 2H), 3.13 (s, 3H), 3.58-3.71 (m, 1H), 4.05 (d, J = 6.08 Hz, 2H), 7.00 (t, J = 6.84 Hz, 1H), 7.20 (t, J = 7.60 Hz, 1H), 7.37 (d, J = 7.60 Hz, 1H), 7.64 (d, J = 7.10 Hz, 1H), 7.98 (d, J = 7.10 Hz, 1H).

### Compound 6E-1 (Yield 53%)

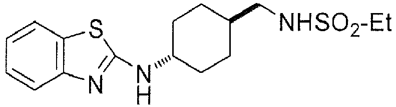

¹H-NMR (DMSO-d₆) δ: 0.93-1.10 (m, 2H), 1.13-1.30 (m, 2H), 1.19 (t, 3H, J = 7.5 Hz), 1.39 (m, 1H), 1.76-1.87 (m, 2H), 2.02-2.14 (m, 2H), 2.79 (t, 2H, J = 6.3 Hz), 2.98 (q, 2H, J = 7.5 Hz), 3.56-3.70 (m, 1H), 6.95-7.05 (m, 2H), 7.20 (t, 1H, J = 7.8 Hz), 7.37 (d, 1H, J = 7.8 Hz), 7.64 (d, 1H, J = 7.5 Hz), 7.92 (d, 1H, J = 7.5 Hz).

### Compound 6E-2 (Yield 68%)

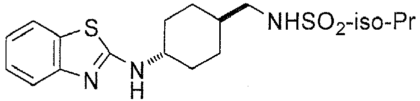

¹H-NMR (DMSO-d₆) δ: 0.96-1.14 (m, 2H), 1.18-1.30 (m, 2H), 1.22 (d, 6H, J = 6.6 Hz), 1.40 (m, 1H), 1.78-1.88 (m, 2H), 2.04-2.14 (m, 2H), 2.81 (t, 2H, J = 6.3 Hz), 3.10-3.20 (m, 1H), 3.58-3.70 (m, 1H), 6.95-7.03 (m, 2H), 7.20 (t, 1H, J = 7.5 Hz), 7.37 (d, 1H, J = 8.1 Hz), 7.64 (d, 1H, J = 7.5 Hz), 7.92 (d, 1H, J = 7.8 Hz).

### Compound 6E-3 (Yield 79%)

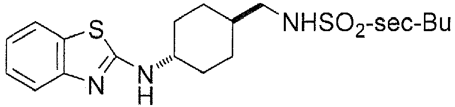

¹H-NMR (DMSO-d₆) δ:0.95 (td, J = 7.4, 2.0 Hz, 3H), 0.98-1.08 (m, 2H), 1.17-1.27 (m, 2H), 1.22 (dd, J = 6.3, 5.8 Hz, 3H), 1.32-1.45 (m, 2H), 1.80-1.96 (m, 4H), 2.05-2.11 (m, 2H), 2.80 (t, J = 6.3 Hz, 2H), 2.89-2.95 (m, 1H), 3.59-3.66 (m, 1H), 6.64 (s, 1H), 6.97-7.01 (m, 1H), 7.16-7.20 (m, 1H), 7.36 (d, J = 8.1 Hz, 1H), 7.63 (d, J = 7.6 Hz, 1H), 7.91 (d, J = 7.6 Hz, 1H).

### Compound 6E-4 (Yield 70%)

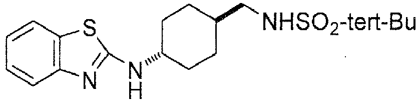

¹H-NMR (DMSO-d₆) δ: 0.97-1.07 (m, 2H), 1.16-1.43 (m, 2H), 1.27 (s, 9H), 1.80-1.85 (m, 2H), 2.06-2.10 (m, 2H), 2.89 (t, J = 6.3 Hz, 2H), 3.59-3.66 (m, 1H), 6.86 (t, J = 5.8 Hz, 1H), 6.99 (t, J = 7.5 Hz, 1H), 7.20 (t, J = 7.5 Hz, 1H), 7.37 (d, J = 8.1 Hz, 1H), 7.63 (d, J = 8.1 Hz, 1H), 7.93 (t, J = 9.9 Hz, 1H).

### Compound 7D

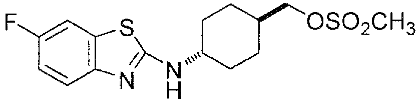

¹H-NMR (CDCl₃) δ: 1.20-1.39 (m, 4H), 1.75-1.87 (m, 1H), 1.92-1.99 (m, 2H), 2.29-2.35 (m, 2H), 3.04 (s, 3H), 3.56-3.65 (m, 1H), 4.10 (d, J = 6.4 Hz, 2H), 5.36 (br s, 1H), 7.03 (ddd, J = 8.8, 8.3, 2.7 Hz, 1H), 7.31 (dd, J = 8.3, 2.5 Hz, 1H), 7.45 (dd, J = 8.8, 4.8 Hz, 1H).

### Compound 7E-1 (Yield 41%)

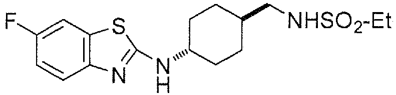

¹H-NMR (DMSO-d₆) δ: 0.94-1.08 (m, 2H), 1.14-1.26 (m, 2H), 1.19 (t, 3H, J = 7.2 Hz), 1.33-1.45 (m, 1H), 1.77-1.86 (m, 2H), 2.03-2.12 (m, 2H), 2.76-2.82 (m, 2H), 2.98 (q, 2H, J = 7.2 Hz), 3.52-3.68 (m, 1H), 6.97-7.06 (m, 2H), 7.34 (dd, 1H, J = 8.4, 4.8 Hz), 7.56 (dd, 1H, J = 8.4, 2.4 Hz), 7.91 (d, 1H, J = 7.6 Hz).

### Compound 7E-2 (Yield 53%)

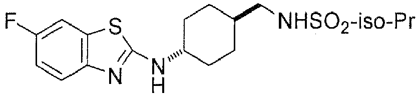

¹H-NMR (DMSO-d₆) δ: 0.92-1.09 (m, 2H), 1.11-1.28 (m, 2H), 1.22 (d, 6H, J = 6.9 Hz), 1.38 (m, 1H), 1.75-1.82 (m, 2H), 2.02-2.12 (m, 2H), 2.81 (t, 2H, J = 6.3 Hz), 3.10-3.20 (m, 1H), 3.52-3.67 (m, 1H), 6.98 (t, 1H, J = 6.0 Hz), 7.03 (td, 1H, J = 9.6, 2.7 Hz), 7.34 (dd, 1H, J = 9.0, 5.1 Hz), 7.57 (dd, 1H, J = 9.0, 2.7 Hz), 7.91 (d, 1H, J = 7.2 Hz).

### Compound 7E-3 (Yield 66%)

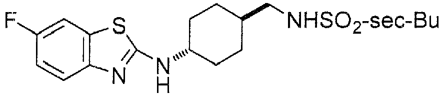

¹H-NMR (DMSO-d₆) δ: 0.95-1.09 (m, 2H), 0.98 (t, J = 7.6 Hz, 3H), 1.16-1.30 (m, 2H), 1.23 (d, J = 6.9 Hz, 3H), 1.35-1.47 (m, 2H), 1.79-1.95 (m, 3H), 2.06-2.13 (m, 2H), 2.82 (t, J = 6.3 Hz, 2H), 2.90-2.97 (m, 1H), 3.58-3.67 (m, 1H), 6.98-7.09 (m, 2H), 7.37 (dd, J = 8.7, 4.9 Hz, 1H), 7.59 (dd, J = 8.7, 2.7 Hz, 1H), 7.93 (d, J = 7.4 Hz, 1H).

### Compound 7F-4 (Yield 54%)

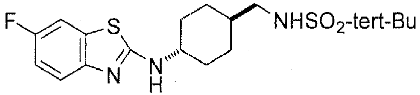

¹H-NMR (DMSO-d₆) δ: 0.92-1.10 (m, 2H), 1.12-1.25 (m, 2H), 1.27 (s, 9H), 1.37 (m, 1H), 1.76-1.84 (m, 2H), 2.02-2.12 (m, 2H), 2.89 (t, 2H, J = 6.0 Hz), 3.50-3.66 (m, 1H), 6.87 (t, 1H, J = 5.7 Hz), 7.03 (dd, 1H, J = 8.7, 2.7 Hz), 7.32-7.37 (m, 1H), 7.58 (dd, 1H, J = 8.7, 2.7 Hz), 7.92 (d, 1H, J = 7.2 Hz).

### Industrial Applicability

A process for the preparation of the present invention can be used to obtain Compound (I) effectively.

## Claims

1. A process for the preparation of a compound of formula (I): 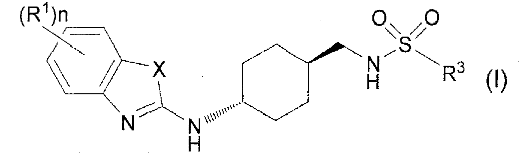
wherein R¹ is each independently halogen, C1-C6 alkyl, C1-C6 alkoxy, C1-C6 haloalkyl, C1-C6 haloalkoxy or C1-C6 alkylcarbonyl,
R³ is C1-C6 alkyl; C3-C8 cycloalkyl; or phenyl optionally substituted with one or more substituents selected from the group consisting of halogen, C1-C6 alkyl; C1-C6 alkoxy, C1-C6 haloalkyl and C1-C6 haloalkoxy,
n is an integer of 0 to 2,
X is S atom or O atom,
its salt or solvate thereof, **characterized by** reacting a compound of formula (IV): 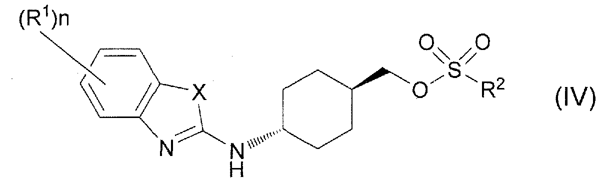
wherein R¹, n and X have the same meaning as defined above, and
R² is C1-C6 alkyl; C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro,
with a compound of formula (V): 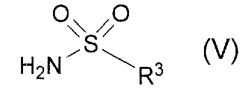 wherein R³ has the same meaning as defined above.

2. The process for the preparation of Claim 1, **characterized by** reacting a compound of formula (IV) with a compound of formula (V) in the presence of a base.

3. The process for the preparation of Claim 2, wherein the base is alkali metal alkoxide or inorganic carbonate.

4. The process for the preparation of Claim 3, wherein the base is potassium tert-butoxide, sodium tert-butoxide, sodium methoxide, sodium ethoxide, sodium pentoxide, sodium carbonate, potassium carbonate, calcium carbonate or cesium carbonate.

5. The process for the preparation of Claim 2, **characterized by** reacting in the polar solvent.

6. The process for the preparation of Claim 5, wherein the polar solvent is one or more solvents selected from the group consisting of methanol, ethanol, isopropanol, n-propanol, tert-butanol, n-butanol, s-butanol, N, N-dimethylformamide, N, N-dimethylacetoamide, N-methylpyrrolidone, 1, 3-dimethyl-2-imidazolidinone, ethyl acetate, propyl acetate, tetrahydrofuran, 2-methyl tetrahydrofuran, dimethylsulfoxide, acetonitrile, propyonitrile, acetone, methylethylketone and methylisobutylketone.

7. The process for the preparation according to any one of claims 1 to 6, wherein R² is methyl.

8. The process for the preparation according to any one of claims 1 to 7, including the process for the preparation of a compound of formula (IV): 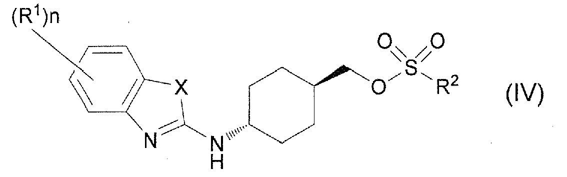
wherein R¹ is each independently halogen, C1-C6 alkyl, C1-C6 alkoxy, C1-C6 haloalkyl, C1-C6 haloalkoxy or C1-C6 alkylcarbonyl,
R² is C1-C6 alkyl; C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro,
n is an integer of 0 to 2,
X is S atom or O atom,
its salt or solvate thereof,
comprising reacting a compound of formula (II): 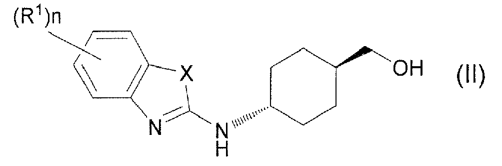
wherein R¹, n and X have the same meaning in a compound of formula (IV), with a compound of formula (III):
R²-SO₂-Y
wherein R² has the same meaning in a compound of formula (IV), and
Y is a leaving group.

9. The process for the preparation of Claim 8, **characterized by** reacting a compound of formula (II) with a compound of formula (III) in the presence of a base.

10. The process for the preparation of Claim 9, wherein the base is an organic base.

11. The process for the preparation of Claim 10, wherein the base is triethylamine, dimethylaminopyridine, diazabicycloundecene, diisopropylethylamine, N-methyl imidazole or N-methylmorpholine.

12. The process for the preparation according to any one of claims 9 to 11, **characterized by** using 2mol to 5mol equivalents of the base to the compound (II).

13. The process for the preparation of Claim 8, **characterized by** reacting a compound of formula (II) with a compound of formula (III) in one or more solvents selected from the group consisting of polar solvent, toluene and dichloromethane.

14. The process for the preparation of Claim 14, wherein the polar solvent is one or more polar solvents selected from the group consisting of N, N-dimethylformamide, N, N-dimethylacetamide, N-methylpyrrolidone, 1, 3-dimethyl-2-imidazolidinone, ethyl acetate, propyl acetate, cyclopentylmethylether, tetrahydrofuran, 2-methyl tetrahydrofuran, dimethylsulfoxide, acetonitrile, propionitrile and methyl isobutyl ketone.

15. The process for the preparation according to any one of claims 8 to 14, **characterized by** not isolating nor purifying a compound of formula (IV), its salt or solvate thereof.

16. The process for the preparation according to any one of claims 8 to 15, **characterized by** using 2mol to 5mol equivalents of the compound (III) to the compound (II).

17. The process for the preparation of a compound of formula (I): 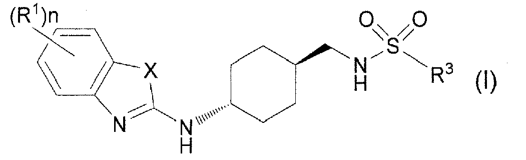
wherein R¹ is each independently halogen, C1-C6 alkyl, C1-C6 alkoxy, C1-C6 haloalkyl, C1-C6 haloalkoxy or C1-C6 alkylcarbonyl,
R³ is C1-C6 alkyl; C3-C8 cycloalkyl; or phenyl optionally substituted with one or more substituents selected from the group consisting of halogen, C1-C6 alkyl; C1-C6 alkoxy, C1-C6 haloalkyl and C1-C6 haloalkoxy,
n is an integer of 0 to 2, and
X is S atom or O atom,
it salt or solvate thereof,
**characterized by** reacting a compound of formula (II): 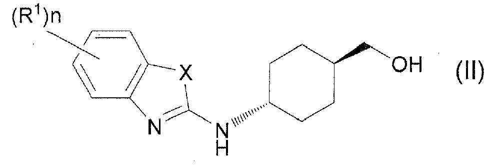
wherein R¹, n and X have the same meaning in a compound of formula (I), with a compound of formula (III):
R²-SO₂-Y
wherein R² is C1-C6 alkyl; C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro,
Y is a leaving group,
to obtain a compound of formula (IV): 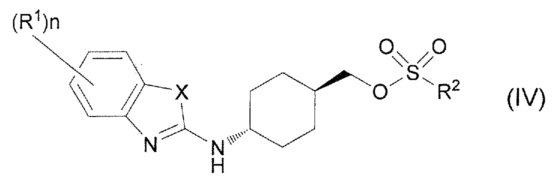 wherein R¹, R², n and X have the same meaning in a compound of formula (II) or (III),
and reacting the obtained compound (IV) with a compound of formula (V): 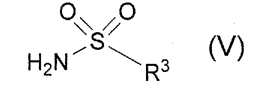 R³ has the same meaning in a compound of formula (I).

18. A compound of formula (II): 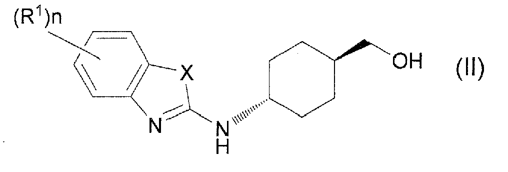
wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2, and
X is S atom or O atom,
its salt or solvate thereof.

19. The compound, its salt or solvate thereof of Claim 18,
wherein R¹ is fluorine or chlorine, and
n is an integer of 0 or 1.

20. A compound of formula (IV): 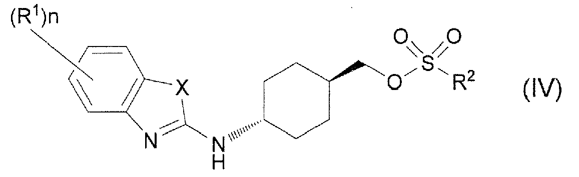
wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2,
X is S atom or O atom, and
R² is C1-C6 alkyl; C1-C6 haloalkyl or phenyl optionally substituted with one or more substituents selected from the group consisting of halogen, C1-C6 alkyl, C1-C6 alkoxy, C1-C6 haloalkyl, C1-C6 haloalkoxy and nitro,
its salt or solvate thereof.

21. The compound, its salt or solvate thereof of Claim 20,
wherein R¹ is fluorine or chlorine,
n is an integer of 0 or 1.

22. The compound, its salt or solvate thereof of Claim 20, wherein R² is methyl.

23. A process for the preparation of a compound of formula (IV): 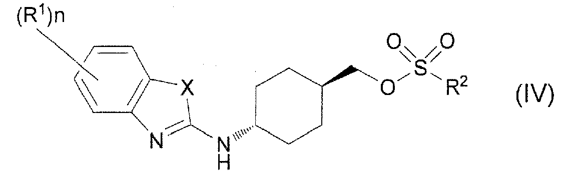
wherein R¹ is each independently halogen, C1-C6 alkyl, C1-C6 alkoxy, C1-C6 haloalkyl, C1-C6 haloalkoxy or C1-C6 alkylcarbonyl,
R² is C1-C6 alkyl; C1-C6 haloalkyl; or phenyl optionally substituted with one or more substituents selected from the group consisting of halogen, C1-C6 alkyl, C1-C6 alkoxy, C1-C6 haloalkyl, C1-C6 haloalkoxy and nitro,
n is an integer of 0 to 2, and
X is S atom or O atom,
its salt or solvate thereof, **characterized by** reacting a compound of formula (II): 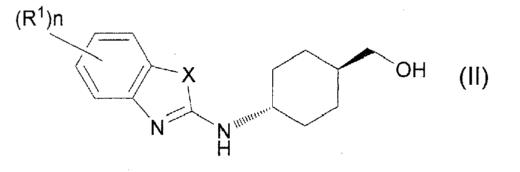
wherein R¹ and n have the same meaning in a compound of formula (IV), with a compound of formula (III):
R²-SO₂-Y
wherein R² has the same meaning in a compound of formula (IV), and
Y is a leaving group.

24. The process for the preparation of Claim 23, **characterized by** using 2mol to 5mol equivalents of the compound (III) to the compound (II).

25. The process for the preparation of Claims 23 or 24, **characterized by** reacting a compound of formula (II) with a compound of formula (III) in the presence of 2mol to 5mol equivalents of the base to the compound (II).

26. A process for the preparation of a compound of formula (II): 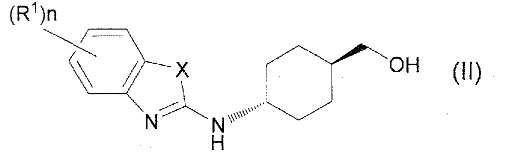
wherein R¹ is each independently halogen, C1-C6 alkyl, C1-C6 alkoxy, C1-C6 haloalkyl, C1-C6 haloalkoxy or C1-C6 alkylcarbonyl,
n is an integer of 0 to 2, and
X is S atom or O atom,
its salt or solvate thereof, **characterized by** reacting a calcium chloride and NaBH₄ with a compound of formula (B): 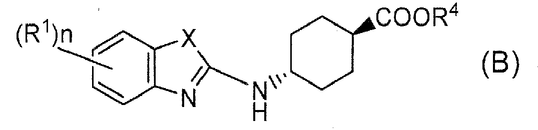 wherein R¹, X and n have the same meaning as defined above, R⁴ is ester residue.
